# EUROPEAN PATENT SPECIFICATION

(11) **EP 3 986 338 B1**
(45) Date of publication and mention of the grant of the patent: **07.01.2026**
(21) Application number: 20733973.0
(22) Date of filing: 18.06.2020
(51) Int. Cl.: A61F 2/78, A61F 2/80

(54) **PROSTHETIC COVER**
PROTHETISCHE ABDECKUNG
BONNET PROTHÉTIQUE

(30) Priority: 18.06.2019 SE 1950750
(43) Date of publication of application: 27.04.2022
(73) Proprietor: C Lindhextend AB, 302 41 Halmstad (SE)
(72) Inventor: LINDHE, Christoffer, 302 91 Halmstad (SE)
(74) Representative: Ström & Gulliksson AB
(86) International application number: PCT/EP2020/067024
(87) International publication number: WO 2020/254529

(56) References cited:
- WO-A1-2017/062690
- WO-A2-2009/017762
- CN-B- 101 820 835
- US-A1- 2007 225 824

## Description

### Technical Field of the Invention

The invention relates to a cover for covering an amputation stump.

### Background

A prosthetic member typically comprises a socket in which an amputation stump is to be inserted. The socket is moulded and cast after the shape of the amputation stump. The socket is made of a rigid and hard material, such as a composite material or a thermoplastic. For the past decades, amputees have been using a tube-like, sock-shaped cover (also referred to as a liner) consisting of a silicone, gel or fabric material to cover the amputation stump beneath the socket of a prosthesis. The prosthetic cover provides additional comfort for and protection of the amputation stump. The prosthetic cover is rolled onto the amputation stump and subsequently the socket of the prosthesis member is fitted onto the stump.

However, when using a prosthesis, the skin of the amputation stump is subjected to shear and pressure, which may cause sore areas and/or abrasions. In addition, amputees often perceive the area of the stump that is in contact with or close to the prosthesis as very warm. This causes discomfort for the user. Even though a prosthetic cover may shield the residual limb from such conditions, the environment beneath the prosthesis and the cover is prone to become warm and humid. A high relative humidity in turn causes the skin to become even more sensitive and vulnerable. Hence, despite that a cover protects the amputation stump, it still may cause an unfavourable environment for the residual limb.

An amputation stump being part of a leg is subjected to high load, supporting the weight of the body of the amputee. Specifically, the bottom part of the amputation stump is subjected and exposed to a high pressure and load. During the time period after the amputation, the stump is especially sensitive. Therefore, it may take up to several years before the patient is able to wear his or her prosthesis for a full day. The amputation stump needs to be trained to be adapted to the high load and to the environment inside the prosthesis before the prosthesis can be used during all hours of the day when the amputee is awake.

CN101820835 and WO 2009/017762 disclose a liner that is suitable between the limb and another prosthetic or orthopedic device.

An object of the present invention is to overcome these problems.

### Summary of the Invention

The above and other objects of the invention are achieved, in full or at least in part, by a tube-shaped cover as set out in claim 1.

According to this claim the above object is achieved by a tube-shaped cover for covering an amputation stump, wherein the cover has at least one open end for introduction of said amputation stump, and wherein the cover comprises an elastic textile material in the shape of a tube; a friction increasing material arranged to be in contact with the amputation stump; and a moisture absorbing and/or moisture transferring material; characterised in that the tube-shaped cover further comprises a material impermeable to air, wherein the material impermeable to air constitutes an outer layer of the cover, wherein the material impermeable to air covers the elastic textile material or the moisture absorbing and/or moisture transferring material. The material impermeable to air may also be referred to as an air-tight material. One advantage of a cover having an outer layer comprising a material impermeable to air is that such an outer layer protects the inner layer(s) from wear.Preferably, the moisture absorbing and/or moisture transferring material is a material that is moisture absorbing or a material that is moisture absorbing and moisture transferring. By absorbing moisture, the relative humidity formed between the stump and the socket of the prosthesis is reduced, which decreases the risk of the user getting abrasions and wounds from the wear from a prosthetic socket.

Hence, in the embodiments presented herein, the moisture absorbing and/or moisture transferring material preferably is a material that is moisture absorbing or a material that is moisture absorbing and moisture transferring.

Specifically, the cover is for use in or together with a prosthetic socket.

Such a tube-shaped cover will enable an amputee to wear a prosthesis with a decreased risk of problems such as blemishes and other painful conditions. Further, such a cover will cause compression of the soft parts of the amputation stump, which favours the distribution of the pressure and load from the prosthesis. In addition, a cover according to the present teaching decreases the relative humidity formed between the stump and the socket of the prosthesis. These advantages will facilitate the amputee to use his or her prosthesis during all awaken hours after only a couple of months training.

In addition, the decreased relative humidity results in a more comfortable experience when using the cover together with a prosthetic socket. The lowered relative humidity results in that the perceived temperature within the socket is maintained at a comfortable level.

The cover is compatible with prostheses having variable fitting mechanisms, such as a vacuum system, a suction system, a lanyard system or a shuttle lock system.

The cover may have the same width or diameter throughout its entire length.

The cover may have different diameters along its length. For example, one open end may have a width or diameter smaller than the diameter of the other end of the cover. Thus, the cover may have a shape similar to a truncated cone. Preferably, the proximal end, i.e. the end intended to be closest to the torso of the patient, of the cover is wider (has larger diameter) than the distal end, i.e. the end intended to be closest to the end of the amputation stump, of the cover. The skilled person realizes that the exact dimensions of a cover according to the present teaching preferably are individually adjusted to fit the amputation stump.

In one example of a cover according to the present teaching, the material impermeable to air covers the moisture absorbing and/or moisture transferring material layer. One advantage of a cover having an outer layer comprising a material impermeable to air is that such an outer layer protects the inner layer(s) from wear.

The elastic textile material of a cover as disclosed herein has a controlled elasticity, which forms a cover with compressive properties. This in turn enables the compression of soft parts of the amputation stump. When the soft parts are compressed, the load of the prosthetic socket is more evenly distributed around the amputation limb, which decreases the risk of abrasions and wounds. It will also make it overall more comfortable to wear the prosthesis.

The elastic textile material in covers according to this teaching may be a compressive or a stretch-limited textile. Such a material will cause compression of the soft parts of the amputation stump, which favours the distribution of the pressure and load from the prosthesis.

The elastic textile material may have an elasticity of between 10 and 600%, such as 50 to 550%, such as 100 to 500%, such as 150 to 450%, such as 200 to 425%, preferably 300 to 400% to form a compressive cover configured to compress the soft tissue of the limb.

The elastic textile material may be acrylic, alpaca, angora, bamboo, cotton, elastane, lycra, hemp, modal, nylon, polyester (such as Coolmax), and/or wool (such as Merino Wool or cashmere wool).

The elastic textile material may be a textile, a fabric, a woven material, such as a silicone woven material made of silicone threads, or a non-woven material.

The friction increasing material in covers according to this teaching may be arranged as stripes, dots, rings and/or figures or as circumferential rings along the surface of the cover.

The friction increasing material in covers according to this teaching are arranged on the surface of the cover intended to be in contact with the amputation stump. i.e. the inside surface of the cover.

The friction increasing material may be silicone, rubber, gel and/or polyurethane (PUR) or any other material which is compatible with skin and which increases the friction between the skin and the cover.

The moisture absorbing and/or moisture transferring material in covers according to this teaching may be a textile, a fabric, a non-woven material, a woven material, a gel and/or a hydrogel or a combination thereof.

The moisture absorbing and/or moisture transferring material helps decrease the relative humidity formed between the stump and the socket of the prosthesis.

High relative humidity is an effect of the tight fitting between the stump and socket, and also the materials used in conventional covers for the stump known in the art. For instance, many conventional covers comprise a compressive material that is not moisture absorbing, resulting in that moisture is not absorbed properly from the stump, which causes the relative humidity adjacent to the skin to increase. Other covers are made of silicone provided with textile on an internal side facing the stump to decrease friction since the friction between the silicone and the skin is too high. Nor this solution absorbs enough moisture to make sure that the relative humidity at the skin surface is kept sufficiently low.

Thus, preferably, a cover according to the present teaching comprises a moisture absorbing material.

A moisture transferring material may also be used, since such a material transports the moisture away from the skin, thus reducing the humidity.

Examples of moisture absorbing and/or moisture transferring materials include wool and silica gels, membranes such as expanded polytetrafluoroethene (ePTFE, such as GoreTex^{®}). Suitable membrane materials have a porous fibre structure, enabling them to be permeable and to transfer moisture, but also to be water impermeable.

Further examples of the moisture absorbing and/or moisture transferring material are acrylic, alpaca, angora, bamboo, cotton, elastane, lycra, hemp, modal, nylon, polyester (such as Coolmax), and/or wool (such as Merino Wool or cashmere wool). Materials such as non-woven materials, wool, silica gels, textiles, fabrics, woven materials, hydrogels or a combination thereof may also be used as the moisture absorbing and/or transferring material.

Preferably, the moisture absorbing material is a super absorbent. The super absorbent may be a cross-linked acrylate copolymer, partially neutralised to its sodium salt.

A silica gel which absorbs moisture may also be used as the moisture absorbing material.

If a natural material is more preferred, wool is preferable since it both transports and absorbs moisture.

The use of moisture absorbing and/or moisture transferring material causes the relative humidity inside the prosthetic socket during use to decrease to levels as low as 50-60%. When using prosthetic covers known in the art beneath (i.e. inside) a prosthetic socket, the relative humidity often quickly, such as within only 15-20 minutes, increases to levels as high as 100%. Human skin is sensitive to moisture, and hence, the resistance of the skin is significantly reduced at high humidity levels. However, at a relative humidity of 50-60%, the skin is much more resistant. An environment having a relative humidity of 100% is also experienced as very hot and uncomfortable, while levels at 50-60% are not perceived as such.

By decreasing the relative humidity in the environment which the skin is exposed to inside the socket, the risk of the user getting abrasions and wounds from the wear from the prosthetic socket is decreased. In addition, the experience for the user is more comfortable since a cover disclosed herein decreases the relative humidity inside the socket.

These effects may facilitate a recently amputated user to be able to use his/her prosthesis for longer periods sooner than when using covers presently available on the market. Further, decreasing the risk of formation of wounds on the stump is also important for users having additional conditions. For example, a user also suffering from diabetes is more prone to bacterial infections caused by wounds formed on the amputation stump.

The moisture absorbing and/or moisture transferring material layer may cover at least part of the elastic textile material.

The moisture absorbing and/or moisture transferring material may be arranged in a uniformly distributed layer around the cover.

The moisture absorbing and/or moisture transferring material may be arranged in a non-uniformly distributed layer around the cover.

The moisture absorbing and/or moisture transferring material may be arranged as a layer having a thickness of between 1 and 10 mm, such as 1 and 8 mm, preferably between 1 and 4 mm, and most preferred 2 to 3 mm.

Preferably, the material referred to herein as a moisture absorbing and/or moisture transferring material is a moisture absorbing material.

As explained above, in some embodiments, the moisture absorbing and/or moisture transferring material and the elastic textile material are the same material.

The cover further comprises a material impermeable to air. The material impermeable to air may also be referred to as an air-tight material.

The material impermeable to air constitutes an outer layer of the cover. One advantage of a cover having an outer layer comprising a material impermeable to air is that such an outer layer protects the inner layer(s) from wear.

The material impermeable to air (i.e. the air-tight material) may be located on the outside, i.e. the surface facing away from the amputation stump, of the elastic textile material. The material impermeable to air may cover the elastic textile material.

The material impermeable to air (i.e. the air-tight material) may be located on the outside, i.e. the surface facing away from the amputation stump, of the moisture absorbing and/or moisture transferring material. The material impermeable to air may cover the moisture absorbing and/or moisture transferring material.

Thus, the material impermeable to air covers the elastic textile material or the moisture absorbing and/or moisture transferring material.

The material impermeable to air may have such properties that moisture may permeate through the material impermeable to air, while air may not permeate the material impermeable to air. Hence, moisture can be transported out from the inside of the cover thus decreasing the humidity inside the prosthetic socket, while air cannot penetrate through the layer of the material impermeable to air.

Preferably, the material impermeable to air is air-tight but at the same time moisture absorbing and/or moisture transferring, which will create a micro climate as described above.

Preferably, the material impermeable to air is air-tight but at the same time moisture absorbing, which will create a micro climate as described above.

Preferably, the material impermeable to air is air-tight but at the same time moisture transferring, which will create a micro climate as described above.

In covers according to the present teaching, the material impermeable to air may be a type of plastic, preferably a hydrophilic copolymer. Most preferably, the material impermeable to air is a polyether based thermoplastic polyurethane (TPU). One example of a thermoplastic polyurethane is "Respire".

The material impermeable to air may be selected from the group consisting of a thermoplastic elastomer, a thermoset elastomer, and mixtures thereof.

Moreover, the material impermeable to air may be a silicone, a urethane, a block copolymer and/or a block copolymer and mineral oil gel composition. The block copolymer may be a styrene-ethylene/butylene-styrene block copolymer, a styrene-ethylene/propylene block copolymer and/or a styrene isoprene/butadiene block copolymer.

According to one embodiment, the moisture absorbing and/or moisture transferring material is a moisture absorbing material.

According to one embodiment, the cover has two open ends. Such a cover (also referred to as a sleeve) may be threaded onto an amputation stump and enclose for instance a knee or an elbow.

Such a sleeve (a cover having two open ends) may be configured to create a seal around an interface between an upper edge of a prosthetic socket and an amputation stump.

Such a sleeve (a cover having two open ends) may be configured to overlap and seal the interface between an upper edge of a prosthetic socket and a cover as disclosed herein.

Such a cover having two open ends (a sleeve) may have the same width or diameter throughout its entire length.

Such a cover having two open ends (a sleeve) may have different diameters along its length. For example, one open end may have a width or diameter smaller than the diameter of the other open end of the cover having two open ends (a sleeve). Thus, the cover having two open ends (a sleeve) may have a shape similar to a truncated cone. Preferably, the proximal end, i.e. the end intended to be closest to the torso of the patient, of the cover having two open ends (a sleeve) is wider (has larger diameter) than the distal end, i.e. the end intended to be closest to the end of the amputation stump, of the cover having two open ends (a sleeve). The skilled person realizes that the exact dimensions of a cover having two open ends (a sleeve) according to the present teaching preferably are individually adjusted to fit the amputation stump.

According to one embodiment, the cover has one open end for introduction of said amputation stump and one closed end opposite said open end.

According to another embodiment, the cover has one open end for introduction of said amputation stump and one closed end opposite said open end. During use, an amputation stump is inserted into the open end. Such a cover may be used together with e.g. a leg or foot prosthesis. Thus, in this embodiment, the cover is used to cover the amputation stump beneath (i.e. inside) the socket of a prosthesis, i.e. as a liner. The cover creates a better micro climate for the amputation stump within the cover and inside a prosthetic socket. The cover creates an environment with lower humidity as described above, which causes possible sores and abrasions to heal better and more quickly than in a climate with higher humidity. Hence, the amputee can use his or her prosthesis even if the stump is sore and in a delicate condition.

In other words, a cover having one open end for introduction of said amputation stump and one closed end opposite said open end is for use as a cover that covers the amputation stump beneath (i.e. inside) the socket of a prosthesis, i.e. as a liner.

A cover having one open end and one closed end may have the same width or diameter throughout its entire length.

A cover having one open end and one closed end may have different diameters along its length. For example, the open end may have a width or diameter larger than the diameter close to the closed end of the cover. Thus, a cover having one open end and one closed end may have a shape similar to a truncated cone. Preferably, the proximal end, i.e. the open end, of the cover may be wider (have larger diameter) than the distal end, i.e. the closed end, of the cover. Furthermore, the closed end of the cover may have a substantially rounded shape, such as to fit the shape of an amputation stump. The skilled person realizes that the exact dimensions of a cover according to the present teaching preferably are individually adjusted to fit the amputation stump.

A cover having one open end and one closed end may also have a seal member attached to the outside of the cover. The seal member may comprise at least one annular flange extending from the cover. The flange aids the formation of vacuum or suction suspension between the cover and the socket and also prevents air from entering in between the cover and the socket during use. The seal member may be made from any material suitable for sealing, such as silicone, rubber or other materials impermeable to air, such as a polymer or a co-polymer.

The skilled person realises that one and same material may simultaneously be elastic, friction increasing, moisture absorbing and/or moisture transferring, and/or impermeable to air. Preferably, the moisture absorbing and/or moisture transferring material is a material that is moisture absorbing or a material that is moisture absorbing and moisture transferring.

Thus, a material may simultaneously be elastic and friction increasing. Examples of such materials include silicone and rubber.

Thus, a material may simultaneously be elastic and moisture absorbing and/or moisture transferring. Examples of such materials include woven materials such as woven wool and woven super absorbent fibres.

Thus, a material may simultaneously be elastic, friction increasing and moisture absorbing and/or moisture transferring. Examples of such materials include hydrophilic plastic materials, such as a woven hydrophilic plastic material.

In one version, a cover according to the present teaching comprises one layer of an elastic textile material, the elastic textile material being a moisture absorbing and/or moisture transferring material and being a friction increasing material, and an outer layer comprising a material impermeable to air. Such a cover will be perceived as being made from two layers, wherein the outer layer comprises a material impermeable to air. One advantage of a cover having an outer layer comprising a material impermeable to air is that such an outer layer protects the inner layer(s) from wear.

Another version of a cover according to the present teaching comprises one layer of an elastic textile material, the elastic textile material being a moisture absorbing and/or moisture transferring material, wherein the elastic textile material has a friction increasing material located on the surface intended to be in contact with an amputation stump. The cover further comprises an outer layer comprising a material impermeable to air located on the surface facing away from the amputation stump.

Another version of a cover according to the present teaching comprises a first layer of an elastic textile material, the elastic textile material being a friction increasing material, and a second layer being impermeable to air and being moisture absorbing and/or moisture transferring located on the surface of the first layer facing away from the amputation stump.

In yet another version of a cover according to the present teaching comprises a first layer of an elastic textile material, the elastic textile material having a friction increasing material located on the surface intended to be in contact with an amputation stump, and a layer being impermeable to air and being moisture absorbing and/or moisture transferring located on the surface of the first layer facing away from the amputation stump.

A cover according to the present teaching may comprise two, three or four layers.

The skilled person realises that two or three of the materials (elastic textile material, friction increasing material, moisture absorbing and/or moisture transferring material and material impermeable to air) can be incorporated into one layer. The different materials may be incorporated into the same layer by means of e.g. knitting, weaving, laminating, stitching or sewing different materials together.

An elastic textile material and a friction increasing material may be incorporated into one layer.

An elastic textile material and a moisture absorbing and/or moisture transferring material may be incorporated into one layer.

An elastic textile material, a friction increasing material and a moisture absorbing and/or moisture transferring material may be incorporated into one layer.

According to one embodiment, the elastic textile material, the moisture absorbing and/or moisture transferring material, and the friction increasing material are distributed between one, two or three layers forming the cover.

A cover according to the present teaching may comprise an elastic textile material, a friction increasing material, a moisture absorbing and/or moisture transferring material incorporated into the same layer. One advantage of such a cover is that the total thickness of the cover is thinner than a cover comprising several different layers. Such a cover may be perceived as an additional skin.

A cover according to the present teaching may comprise two layers.

In one example of a cover comprising two layers, the first layer is intended to have direct contact with an amputation stump and comprises an elastic textile material, a moisture absorbing and/or moisture transferring material and a friction increasing material; and the second layer covers the first layer and comprises a material impermeable to air. One advantage of a cover having an outer layer comprising a material impermeable to air is that such an outer layer protects the inner layer(s) from wear.

In another example of a cover comprising two layers, the first layer is intended to have direct contact with an amputation stump and comprises an elastic textile material and a friction increasing material; and the second layer covers the first layer and comprises a moisture absorbing and/or moisture transferring material and a material impermeable to air.

In another example of a cover comprising two layers, the first layer is intended to have direct contact with an amputation stump and comprises a moisture absorbing and/or moisture transferring material and a friction increasing material; and the second layer covers the first layer and comprises an elastic textile material and a material impermeable to air.

A cover according to the present teaching may comprise three layers.

In one example of a cover comprising three layers, the first layer is intended to have direct contact with an amputation stump and comprises an elastic textile material and a friction increasing material; the second layer at least partly covers the first layer and comprises a moisture absorbing and/or moisture transferring material; and the third layer covers the second layer and comprises a material impermeable to air.

In another example of a cover comprising three layers, the first layer is intended to have direct contact with an amputation stump and comprises a moisture absorbing and/or moisture transferring material and a friction increasing material; the second layer at least partly covers the first layer and comprises an elastic textile material; and the third layer covers the second layer and comprises a material impermeable to air.

In another example of a cover comprising three layers, the first layer is intended to have direct contact with an amputation stump and comprises a friction increasing material; the second layer at least partly covers the first layer and comprises an elastic textile material and a moisture absorbing and/or moisture transferring material; and the third layer covers the second layer and comprises and a material impermeable to air.

In another example of a cover comprising three layers, the first layer is intended to have direct contact with an amputation stump and comprises a friction increasing material; the second layer at least partly covers the first layer and comprises an elastic textile material; and the third layer covers the second layer and comprises a moisture absorbing and/or moisture transferring material and a material impermeable to air.

In another example of a cover comprising three layers, the first layer is intended to have direct contact with an amputation stump and comprises a friction increasing material; the second layer at least partly covers the first layer and comprises a moisture absorbing and/or moisture transferring material; and the third layer covers the second layer and comprises an elastic textile material and a material impermeable to air.

A cover according to the present teaching may comprise four layers.

In one example of a cover comprising four layers, the first layer is intended to have direct contact with an amputation stump and comprises a friction increasing material; the second layer at least partly covers the first layer and comprises an elastic textile material; the third layer at last partly covers the second layer and comprises a moisture absorbing and/or moisture transferring material; and a fourth layer covers the third layer and comprises a material impermeable to air.

In another example of a cover comprising four layers, the first layer is intended to have direct contact with an amputation stump and comprises a friction increasing material; the second layer at least partly covers the first layer and comprises a moisture absorbing and/or moisture transferring material; the third layer at last partly covers the second layer and comprises an elastic textile material; and a fourth layer covers the third layer and comprises a material impermeable to air.

In another example of a cover comprising four layers, the first layer is intended to have direct contact with an amputation stump and comprises a friction increasing material; the second layer at least partly covers the first layer and comprises a moisture absorbing and/or moisture transferring material; the third layer covers the second layer and comprises a material impermeable to air; and a fourth layer at least partly covers the third layer and comprises an elastic textile material.

In another example of a cover comprising four layers, the first layer is intended to have direct contact with an amputation stump and comprises a friction increasing material; the second layer at least partly covers the first layer and comprises an elastic textile material; the third layer covers the second layer and comprises a material impermeable to air; and a fourth layer at least partly covers the third layer and comprises a moisture absorbing and/or moisture transferring material.

Thus, versions of a cover according to the present teaching may comprise one layer of an elastic textile material and a layer of a moisture absorbing and/or moisture transferring material located on the surface of the elastic textile material facing away from an amputation stump when covered by the cover, wherein the elastic textile material has a friction increasing material located on the surface intended to be in contact with an amputation stump. The material impermeable to air may be incorporated in the moisture absorbing and/or moisture transferring material. Alternatively, an outer layer comprising a material impermeable to air may cover the moisture absorbing and/or moisture transferring material layer, in which case the material impermeable to air is also permeable to moisture.

Thus, further versions of a cover according to the present teaching comprise one layer of a moisture absorbing and/or moisture transferring material and a layer of an elastic textile material located on the surface of the moisture absorbing and/or moisture transferring material facing away from an amputation stump when covered by the cover, wherein the moisture absorbing and/or moisture transferring material has a friction increasing material located on the surface intended to be in contact with an amputation stump. The material impermeable to air may be incorporated in the elastic textile material. Alternatively, an outer layer comprising a material impermeable to air covers the elastic textile material.

According to one embodiment, two or three of the elastic textile material, the friction increasing material, and the moisture absorbing and/or moisture transferring material are incorporated in the same material. The different materials may be incorporated into the same material by means of e.g. knitting, weaving, laminating, stitching or sewing different materials together.

Thus, in some examples, two of the elastic textile material, the friction increasing material, the moisture absorbing and/or moisture transferring material and the material impermeable to air are incorporated in the same material.

The elastic textile material and the friction increasing material may be incorporated in the same material.

The elastic textile material and the moisture absorbing and/or moisture transferring material may be incorporated in the same material.

The elastic textile material and the material impermeable to air may be incorporated in the same material.

The moisture absorbing and/or moisture transferring material and the friction increasing material may be incorporated in the same material.

The moisture absorbing and/or moisture transferring material and the material impermeable to air may be incorporated in the same material.

Thus, in some examples, three of the elastic textile material, the friction increasing material, the moisture absorbing and/or moisture transferring material and the material impermeable to air are incorporated in the same material.

The elastic textile material, the friction increasing material and the moisture absorbing and/or moisture transferring material may be incorporated in the same material.

The elastic textile material, the friction increasing material and the material impermeable to air may be incorporated in the same material.

The friction increasing material, the moisture absorbing and/or moisture transferring material and the material impermeable to air may be incorporated in the same material.

According to one embodiment, the friction increasing material is incorporated in the elastic textile material. In this way, the cover can be thinner than a cover having a separate layer of friction increasing material. Such a cover will not be perceived as warm as a thicker cover. In addition, the contact between the amputation stump and the prosthesis is much better.

According to another embodiment, the moisture absorbing and/or moisture transferring material is incorporated in the elastic textile material. Such a cover will not be perceived as warm as a thicker cover. The moisture absorbing and/or transferring material will transfer moisture from the stump, which creates a cooling effect. Additionally, the humidity level will become lower, which in turn decreases the heat perception within the prosthetic socket. In addition, the contact between the amputation stump and the prosthesis is much better.

The skilled person realises that both the friction increasing material and the moisture absorbing and/or moisture transferring material may be incorporated in the elastic textile material.

The material impermeable to air may be incorporated in the moisture absorbing and/or moisture transferring material. Such a cover is thinner than a cover comprising separate materials, which gives a closer contact between the amputation stump and the socket. In addition, the cover is firmer, which leads to more freedom from play between the cover and the socket. Further, the cover is perceived as an additional protective skin layer around the amputation stump.

The moisture absorbing and/or moisture transferring material may be the same material as the air-tight material. Such a cover is thinner than a cover comprising separate materials, which gives a closer contact between the amputation stump and the socket. In addition, the cover is firmer, which leads to more freedom from play between the cover and the socket. Further, the cover is perceived as an additional protective skin layer around the amputation stump.

According to a further embodiment, the elastic textile material is a textile, a fabric, a woven material, such as a silicone woven material made of silicone threads, or a non-woven material. In some cases, such as when the elastic textile material is a silicone material or a rubber material, such as a woven material made from silicone or rubber material the elastic textile material is also a friction increasing material. Thus, the friction increasing material is evenly distributed throughout the material.

As stated above, in some examles, the outer layer comprising the material impermeable to air covers the moisture absorbing and/or moisture transferring material layer. Such a cover may be used together with a prosthesis that is to be fitted using a vacuum or suction suspension system. The material impermeable to air aids in preventing air from penetrating into the socket and disrupting the vacuum formed therein, which would lead to the prosthesis not being tightly attached to the amputation stump. In addition, the air-tight material protects the inner layers from wear.

If the cover is used together with a prosthesis using a vacuum fastening system, it is important to prohibit air from entering into the socket of the prosthesis. This may be accomplished by a sleeve as explained above and/or a seal member located on the outside of the cover.

Preferably, the material impermeable to air (i.e. the air-tight material) is located on the outside of the elastic textile material.

In one embodiment, the material impermeable to air is a type of plastic, preferably a hydrophilic copolymer. Most preferably, the material impermeable to air is a polyether based thermoplastic polyurethane (TPU). One example of a thermoplastic polyurethane is "Respire".

Preferably, the material impermeable to air is air tight but at the same time permeable for moisture, which will create a micro climate as described above.

The material impermeable to air may be selected from the group consisting of a thermoplastic elastomer, a thermoset elastomer, and mixtures thereof.

Moreover, the material impermeable to air may be a silicone, a urethane, a block copolymer and/or a block copolymer and mineral oil gel composition. The block copolymer may be a styrene-ethylene/butylene-styrene block copolymer, a styrene-ethylene/propylene block copolymer and/or a styrene isoprene/butadiene block copolymer.

The material impermeable to air may have such properties that moisture may permeate through the material impermeable to air, while air may not permeate the material impermeable to air. Hence, moisture can be transported out from the inside of the cover thus decreasing the humidity inside the prosthetic socket, while air cannot penetrate through the layer of the material impermeable to air.

According to one embodiment, the cover is configured to be used together with a prosthetic socket, which is to be suspended to an amputation stump by a vacuum created between the cover and the socket when the amputation stump is introduced into said cover. The vacuum may be created by the tight fitting of the socket to the amputation stump covered by a cover according to the present teaching.

When the cover has been rolled or threaded onto the amputation stump, the stump is inserted and fitted into the socket of a prosthesis. Subsequently, by using a mechanical or electrical pump, often placed on a side of the prosthesis, air is removed to form vacuum between the cover and the socket. The air may be actively removed by the use of a vacuum pump. Current examples include "Harmony" (Ottobock) and "Unity" (Össur). Alternatively, the stump provided with the cover is inserted and fitted into a suction suspension system, and suction suspension holds the prosthesis securely attached to the stump. Current examples include "Iceross Seal-In" (Össur).

According to another embodiment, the moisture absorbing and/or moisture transferring material is arranged around the elastic textile material, wherein a thickness of the moisture absorbing material and/or moisture transferring material at the closed end of the cover is thicker than a thickness of the moisture absorbing material at the rest of the cover. Thus, in this embodiment, the elastic textile material is arranged to be closer to the amputation stump than the moisture absorbing and/or moisture transferring material and the moisture absorbing and/or moisture transferring material covers the elastic textile material. The thicker layer of the moisture absorbing and/or transferring material moderates the load and pressure between the residual limb and the prosthesis. This provides a more comfortable wear for the amputee and reduces the risk of abrasions on the end of the amputation stump.

According to another embodiment, the cover further comprises a cushioning material at the closed end of the cover, configured to moderate the load between the amputation stump and a prosthetic socket. The cushioning material moderates the load between the residual limb and the prosthesis. This provides a more comfortable wear for the amputee and reduces the risk of abrasions on the end of the amputation stump. This is especially convenient for stumps being extra sensitive or for newly amputated patients. How sensitive a stump is varies between different patients.

The cushioning material may be arranged on the inside surface, i.e. the surface intended to face the amputation stump, of the cover.

The cushioning material may be arranged on the outside surface of the cover.

The cushioning material may be arranged both on the inside surface, i.e. the surface intended to face the amputation stump, of the cover and on the outside surface of the cover.

The cushioning material may for instance be the same material as the moisture absorbing and/or moisture transferring material. In such case, the cushion may be arranged as a thicker layer of the moisture absorbing and/or moisture transferring material at the closed end of the cover.

The cushioning material may be any soft material. Further examples are a silicone or a gel.

According to one embodiment, the cover further comprises a threaded portion arranged at the closed end of the cover, wherein the threaded portion is adapted for the fastening of a pin configured to engage in a lock in a prosthetic socket, and optionally a strap configured to guide the pin into the lock; or a strap configured to engage in a lock in a prosthetic socket.

Such a cover may be used together with a pin and lock system or together with a lanyard system to fasten a prosthesis to the cover. Current examples of a pin and lock system include "Iceross Seal-In" (Össur).

The threaded portion may be adapted for the fastening of a pin configured to engage in a lock in a prosthetic socket. Optionally, the threaded portion may also be adapted for the fastening of strap configured to guide the pin into the lock.

The threaded portion arranged at the closed end of the cover may be a female threaded portion adapted for the fastening of a pin comprising a male threaded portion. The male threaded portion of the pin may be screwed into the female threaded portion. Such a cover may be used together with a pin and lock system. A male threaded portion of a pin of e.g. a pin and lock system can be screwed into the female threaded portion arranged at the closed end of the cover. The pin is configured to engage with a lock arranged at the bottom in a prosthetic socket thus securing fastening of the cover in the prosthetic socket.

The lock may be a shuttle lock.

When the cover is used together with a pin and lock system, such as a shuttle lock system, the cover comprises an open end and a female threaded portion arranged at a closed end of the cover. The pin comprises a male threaded portion. The pin is thus removably connectable to the cover. When in use, the cover is mounted onto an amputation stump and the male threaded portion of the pin is screwed into the female threaded portion arranged at a closed end of the cover. The amputation stump covered with the cover is then inserted into a prosthetic socket having a lock in its bottom. The pin engages with the lock to secure the fastening of the cover in the prosthetic socket.

If a strap configured to guide the pin into the lock is present, the cover is mounted onto an amputation stump and the strap is inserted into an opening arranged at the bottom of the socket. By pulling the strap, the amputation stump wearing the cover is guided into the socket and the pin is guided into the lock arranged at the bottom of the socket.

Alternatively, the threaded portion of the cover is adapted for the fastening of a strap configured to engage in a lock in a prosthetic socket. Such a cover may be used with a so called lanyard system. The strap may be fastened to the cover by e.g. a screw, that is screwed into a female threaded portion arranged at the closed end of the cover.

The strap is pulled through a hole in the bottom of a prosthetic socket, the amputation stump wearing the cover is inserted into the socket, and the strap is subsequently securely fastened in for instance a loop, ring or hook on the outer side of the prosthetic socket. The strap may be threaded through the loop, ring or hook and secured to itself by any suitable fastening means. Current examples include "Ice-Lock" (Össur) and KISS lanyard system (Ottobock).

A cover intended to be used with a lanyard system as described above may comprise a further strap arranged near and proximal to the top open end of the cover. The socket of the prosthesis in the lanyard system comprises a slit through which the strap arranged near and proximal to the top open end of the cover may be introduced from the inside of the socket to the outside. This strap also comprises a loop, which is thus moved through the slit and is arranged on the outside of the socket once the strap has been pulled through the slit. The strap arranged at the closed end of the cover comprises at a portion close to the cover a first part of a hook-and-loop system and at a second end a second part of a hook-and-loop system. The bottom of the socket comprises a slit where the strap arranged at the closed end of the cover is inserted from the inside to the outside of the socket. When this strap has been pulled through the slit, it is threaded through the loop of the strap arranged near and proximal to the top open end of the cover. Subsequently, the two ends of the strap arranged at the closed end of the cover may attach to each other by any suitable fastening means, such as "Alpha Lock (WillowWood), whereupon the two parts of the hook-and-loop system engage with each other. The prosthesis has now been fastened to the amputation stump and the cover protects the stump from abrasions and too high humidity during wear.

Thus, a cover according to the present teaching may comprise a first strap arranged at the near and proximal to the top open end of the cover and a second strap arranged at the closed end of the cover, wherein the two straps are configured to form a lanyard system for fastening of a prosthetic socket. Current examples include "Ice-Lock" (Össur) and KISS lanyard system (Ottobock). A threaded portion arranged at the closed end of the cover may be adapted for the fastening of the second strap.

In one embodiment, the cover further comprises a strap arranged proximal to the closed end of said cover, wherein the strap is configured to guide an amputation stump covered by said cover into a prosthetic socket.

The socket of the prosthesis has a slit through which the strap may be introduced from the inside of the socket to the outside. By pulling the strap the amputation stump wearing the cover is guided into the right place in the socket.

The cover may further comprise a second strap at the bottom closed end of the cover.

The first strap may comprise a loop and the second strap may comprise a first and second part of a hook-and-loop system. Further, the second strap may be configured to be threaded through the loop of the first strap such that the first and second part of the hook-and-loop system may engage with each other.

Such a cover may be used with a so-called lanyard system. The cover is fitted onto the amputation stump. Further, the socket of the prosthesis in the lanyard system comprises a first slit through which the first strap may be introduced from the inside of the socket to the outside. The first strap also comprises a loop, which is thus moved through the slit and is arranged on the outside of the socket once the first strap has been pulled through the first slit. The second strap comprises at a portion close to the cover a first part of a hook-and-loop system and at a second end a second part of a hook-and-loop system. The bottom of the socket comprises a second slit where the second strap is inserted from the inside to the outside of the socket. When the second strap has been pulled through the second slit, it is threaded through the loop of the first strap. Subsequently, the two ends of the second strap may attach to each other whereupon the two parts of the hook-and-loop system engage with each other. The prosthesis has now been fastened to the amputation stump and the cover protects the stump from abrasions and too high humidity during wear.

According to one embodiment, the thickness of the cover is between 1 and 10 mm, such as 1.25 and 8 mm, such as 1.5 and 6 mm, preferably 2-5 mm.

The cover may have a length of 10 to 50 cm, such as 20 to 40 cm, preferably approximately 30 cm.

The cover having one open end and one closed end may typically have a length of 10 to 50 cm, such as 20 to 40 cm, preferably approximately 30 cm. The exact length of the cover may be individually adjusted to fit the specific stump.

The cover having two open ends, i.e. a sleeve, may typically have a length of 10 to 70 cm, such as 20 to 60 cm, preferably approximately 40 cm. The exact length of the cover may be individually adjusted to fit the specific stump.

According to a second aspect, there is provided a kit comprising a cover having one open end and one closed end according to the present teaching and a sleeve configured to create a seal between an upper edge of a prosthetic socket and an amputation stump covered by said cover. The sleeve is configured to overlap and seal the interface between an upper edge of a prosthetic socket and a cover as disclosed herein. The sleeve is thus configured to create a seal around an interface between an upper edge of a prosthetic socket and an amputation stump covered by said cover.

The cover and the sleeve may be used separately or jointly together with each other.

A cover according to the present teaching may also be referred to as a prosthetic cover, a liner or a prosthetic liner.

Herein, an amputation stump may also be referred to as a stump, an amputation limb or a residual limb.

Herein, an air-tight material may also be referred to as a material that is impermeable to air.

The term textile is defined herein as being made from fiber, yarn or fabric or a combination of any of fiber, yarn or fabric. A textile can be non-woven or woven.

The term fabric is defined herein as being made from fiber and/or yarn. A fabric can be non-woven or woven. A fabric may also be e.g. knitted.

Thus, the term textile has a broader meaning than the meaning of the word fabric.

Other objectives, features and advantages of the present invention will appear from the following detailed disclosure, from the attached claims, as well as from the drawings. It is noted that the invention relates to all possible combinations of features.

Generally, all terms used in the claims are to be interpreted according to their ordinary meaning in the technical field, unless explicitly defined otherwise herein. All references to "a/an/the [element, device, component, etc.]" are to be interpreted openly as referring to at least one instance of said element, device, component, etc., unless explicitly stated otherwise.

As used herein, the term "comprising" and variations of this term are not intended to exclude other components or integers.

### Brief description of the Drawings

By way of example, embodiments of the present teaching will now be described with reference to the accompanying drawings, in which:
Fig. 1a shows a lateral cross section of one embodiment of a cover according to the present teaching showing an elastic textile material layer (all layers are not shown);
Fig. 1b shows a lateral cross section of one embodiment of a cover according to the present teaching showing an elastic textile material layer and an outer layer of an air-tight material (all layers are not shown);
Fig. 1c shows a lateral cross section of one embodiment of a cover according to the present teaching showing a friction increasing layer, a layer of an elastic textile material and an air-tight material layer (all layers are not shown);
Fig. 1d shows a lateral cross section of one embodiment of a cover according to the present teaching showing a friction increasing layer, a layer of an elastic textile material, a moisture absorbing and/or transferring layer and an air-tight material layer;
Fig. 2a shows a lateral cross section of one embodiment of a cover according to the present teaching having a thicker right hand side than the left hand side of a moisture absorbing and/or transferring material;
Fig. 2b shows a lateral cross section of one embodiment of a cover according to the present teaching having a thicker bottom layer of a moisture absorbing and/or transferring material;
Fig. 2c-d show a lateral cross section of embodiments of a cover according to the present teaching having a cushion formed of a cushioning material 28 at the closed end 22 of the cover.
Fig. 3a-3e show exemplary arrangements of a friction increasing material in different embodiments of a cover according to the present teaching;
Fig. 4 shows an amputation stump covered with a cover introduced and fitted into a hard socket of a prosthesis, such as a prosthetic leg;
Fig. 5 shows an alternative embodiment of the prosthetic cover shaped as a sleeve;
Fig. 6 shows a cover comprising a threaded insert for the connection of a pin; and
Fig. 7a-7d show exemplary arrangements of a seal member on a cover according to the present teaching.

### Detailed Description of Preferred Embodiments

The present teaching relates to a cover, also referred to as a prosthetic cover, a liner or a prosthetic liner, having a tube-like shape and comprising an elastic textile material, a friction increasing material arranged to be in contact with an amputation stump, and a moisture absorbing and/or moisture transferring material. Preferably, the moisture absorbing and/or moisture transferring material is a moisture absorbing material. The cover also includes a material impermeable to air. Thus, the present teaching relates to a tube-shaped cover (10, 20, 30, 40, 50, 60, 70) for covering an amputation stump (1), wherein the cover has at least one open end (11, 21, 31, 41, 51, 61, 71) for introduction of said amputation stump (1), wherein the cover (10, 20, 30, 40, 50, 60, 70) comprises an elastic textile material (13) in the shape of a tube; a friction increasing material (14) arranged to be in contact with the amputation stump (1); and a moisture absorbing and/or moisture transferring material (16); wherein the tube-shaped cover further comprises a material impermeable to air (17), wherein the material impermeable to air (17) constitutes an outer layer of the cover, wherein the material impermeable to air (17) covers the elastic textile material (13) or the moisture absorbing and/or moisture transferring material (16). The amputation stump is also referred to herein as the stump or residual limb.

In the following, the cover is referred to by the reference numbers 10a-d, 20a-d, 30a-e, 40, 50, 60 and 70a-70d.

In short, the disclosed prosthetic cover enables the amputee to wear a prosthesis with a decreased risk of problems such as blemishes and other painful conditions. Further, the cover disclosed herein will cause compression of the soft parts of the amputation stump, which favours the distribution of the pressure and load from the prosthesis. In addition, the cover decreases the relative humidity formed between the stump and the socket of the prosthesis. These advantages will facilitate the amputee to use his or her prosthesis during all awaken hours after only a couple of months training. The cover is compatible with prostheses having variable fitting mechanisms, such as a vacuum system, a suction system, a lanyard system or a shuttle lock system.

The cover may comprise an elastic textile material 13, a moisture absorbing and/or moisture transferring material 16, a friction increasing material 14 and an air-tight material 17, distributed between two, three or four layers forming the cover 10a-d.

Embodiments of the cover according to the present teaching will now be described in relation to the accompanying figures.

Preferably, in the embodiments presented herein, the moisture absorbing and/or moisture transferring material is a material that is moisture absorbing or a material that is moisture absorbing and moisture transferring.

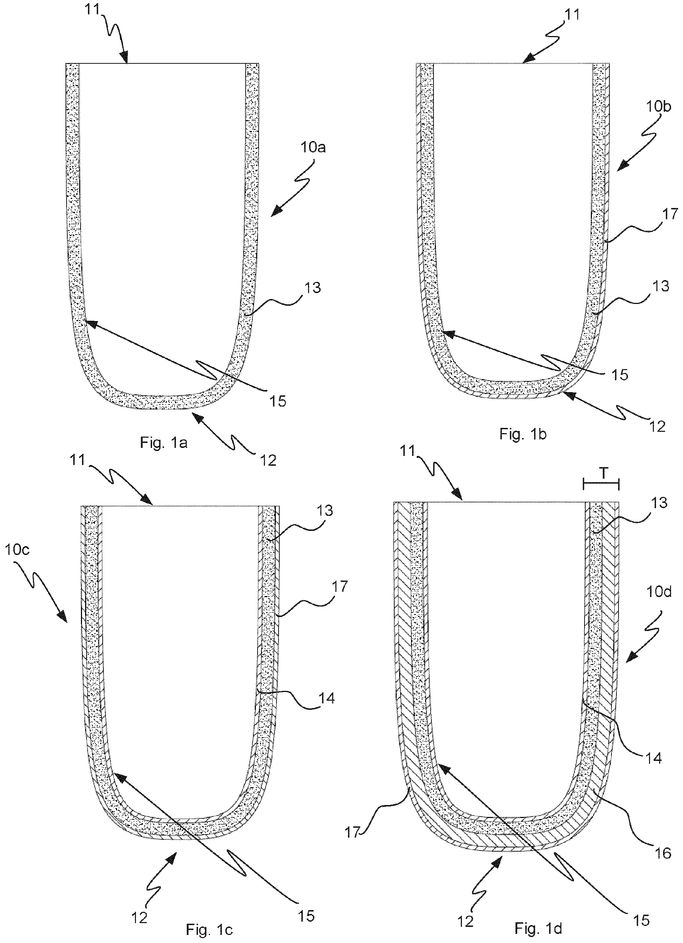

The tube-shaped covers 10a, 10b, 10c, 10d shown in Figs. 1a-1d all comprise an open end 11 and a closed end 12 opposite to the open end 11. During use, an amputation stump is inserted into the open end 11.

A tube-shaped cover as shown in Figs. 1a-1d is for use as covers that covers the amputation stump beneath (i.e. inside) the socket of a prosthesis, i.e. as liners.

A tube-shaped cover as shown in Figs. 1a-1d may have the same width or diameter throughout its entire length.

A tube-shaped cover as shown in Figs. 1a-1d may have different diameters along its length. For example, the open end 11 may have a width or diameter larger than the diameter close to the closed end 12 of the cover 10a, 10b, 10c, 10d. Thus, a tube-shaped cover as shown in Figs. 1a-1d may have a shape similar to a truncated cone. Preferably, the proximal end, i.e. the open end 11, of the cover 10a, 10b, 10c, 10d may be wider (have larger diameter) than the distal end, i.e. the closed end 12, of the cover 10a, 10b, 10c, 10d. Furthermore, the closed end 12 of the cover 10a, 10b, 10c, 10d may have a substantially rounded shape, such as to fit the shape of an amputation stump. The skilled person realizes that the exact dimensions of the cover 10a, 10b, 10c, 10d preferably are individually adjusted to fit the amputation stump.

Fig. 1a shows a lateral cross section of a tube-shaped prosthetic cover 10a comprising an elastic textile material 13 which is also a moisture absorbing and/or a moisture transferring material. In this embodiment, the elastic textile material 13 provides a cover having enough friction to keep the cover tightly arranged on the amputation stump, i.e. the elastic textile material 13 is also a friction increasing material. Since the elastic textile material 13 compresses the stump, the elastic textile material 13 may also have friction increasing properties. The cover 10a shown in Fig. 1a further comprises a material impermeable to air (not shown).

In some embodiments, the elastic textile material 13 is a compressive material, or a stretch-limited material.

In some embodiments, the elastic textile material 13 is a material impermeable to air.

In one embodiment (not shown), the cover comprises an elastic textile material 13, which is also a moisture absorbing and/or a moisture transferring material, and a separate friction increasing material 14.

The friction increasing material 14 is arranged on an interior surface 15 of the cover 10c.

The friction increasing material 14 may cover the entire interior surface 15 of the cover.

The friction increasing material 14 may cover only a part or parts of the interior surface 15 of the cover.

The friction increasing material may have different geometrical forms, such as stripes, rings, circles, rectangles, stars, ovals etc..

The friction increasing material 14 may be evenly spread on the entire interior surface 15.

The friction increasing material 14 may be unevenly spread on the entire interior surface 15, such as being more densely spread on certain parts of the interior surface 15, or being present only in certain parts of the interior surface 15.

Commonly, the friction increasing material 14 is arranged as dots or stripes/circumferential rings on the interior surface 15 of the cover.

The cover 10b shown in Fig. 1b comprises an elastic textile material 13 which is also a moisture absorbing and/or a moisture transferring material, and a layer of an air-tight material 17, also referred to herein as a material impermeable to air. In this embodiment, the elastic textile material 13 provides a cover having enough friction to keep the cover tightly arranged on the amputation stump, i.e. the elastic textile material 13 is also a friction increasing material. The elastic properties of the elastic textile material 13 provides friction between the skin and the cover. In some embodiments, the elastic textile material 13 is a compressive material, or a stretch-limited material. The air-tight material 17 (i.e. the material impermeable to air) covers the elastic textile material 13.

Preferably, the air-tight material 17 is a type of plastic. Preferred plastics are hydrophilic copolymers, such as for instance polyether based thermoplastic polyurethane (TPU). One example of a thermoplastic polyurethane is "Respire" since these materials are impermeable to air and permeable to moisture.

The air-tight material 17 may be selected from the group consisting of a thermoplastic elastomer, a thermoset elastomer, and mixtures thereof.

Moreover, the air-tight material 17 may be a silicone, a urethane, a block copolymer and/or a block copolymer and mineral oil gel composition. The block copolymer may be a styrene-ethylene/butylene-styrene block copolymer, a styrene-ethylene/propylene block copolymer and/or a styrene isoprene/butadiene block copolymer.

The air-tight material 17 may have such properties that moisture may permeate through the air-tight material 17, while air may not permeate the air-tight material 17. Hence, moisture can be transported out from the inside of the cover 10b thus decreasing the humidity inside the prosthetic socket, while air cannot penetrate through the layer of the air-tight material 17.

The use of an air-tight material 17 is preferable when the prosthesis is to be fitted using a vacuum or suction suspension system. When the cover 10 has been rolled or threaded onto the amputation stump, the stump is inserted and fitted into the socket of the prosthesis. Subsequently, by using a mechanical or electrical pump, often placed on a side of the prosthesis, air is removed to form vacuum between the cover 10 and the socket. Alternatively, the stump provided with the cover 10 is inserted and fitted into a suction suspension system, and suction suspension holds the prosthesis securely attached to the stump. The air-tight material 17 achieves the suction suspension. The air-tight material 17 thus aids in preventing air from penetrating into the socket and disrupting the vacuum/suction formed therein, which would lead to the prosthesis not being tightly attached to the amputation stump.

In Fig. 1c, a cover 10c comprising a layer of a friction increasing material 14 is shown. The cover 10c further comprises an elastic textile material 13, which is also a moisture absorbing and/or a moisture transferring material and a layer of an air-tight material 17.

The friction increasing material 14 is arranged on an interior surface 15 of the cover 10c.

The friction increasing material 14 may cover the entire interior surface 15 of the cover.

The friction increasing material 14 may cover only a part or parts of the interior surface 15 of the cover.

The friction increasing material may have different geometrical forms, such as stripes, rings, circles, rectangles, stars, ovals etc..

The friction increasing material 14 may be evenly spread on the entire interior surface 15.

The friction increasing material 14 may be unevenly spread on the entire interior surface 15, such as being more densely spread on certain parts of the interior surface 15, or being present only in certain parts of the interior surface 15.

Preferably, the friction increasing material is present as dots or stripes/circumferential rings on the interior surface 15 of the cover.

In one embodiment (not shown), the cover comprises an elastic textile material 13 and a separate layer of a moisture absorbing and/or moisture transferring material 16. In this embodiment, the elastic textile material 13 provides a cover having enough friction to keep the cover tightly arranged on the amputation stump, i.e. the elastic textile material 13 is also a friction increasing material. In some embodiments, the elastic textile material 13 is a compressive material, or a stretch-limited material. The moisture absorbing and/or moisture transferring material 16 may also be an air-tight material.

In Fig. 1d, a cover 10d according to the present teaching is shown. The cover 10d comprises a layer of an elastic textile material 13. Further, it comprises a layer of a friction increasing material 14 arranged on an interior surface 15 of the cover 10d and a separate layer of a moisture absorbing and/or moisture transferring material 16 as well as a layer of an air-tight material 17. Details of the different layers are as explained above.

The cover 10 shown in Fig. 1d has a thickness T, which is between 1 and 6 mm. Preferably, the thickness T is between 2 and 5 mm.

In one embodiment (not shown), the cover comprises a layer of an elastic textile material 13. Further, it comprises a layer of a friction increasing material 14 arranged on an interior surface 15 of the cover 10d and a separate layer in which a moisture absorbing and/or moisture transferring material as well as a layer of an air-tight material are incorporated. Thus, in this embodiment the moisture absorbing and/or moisture transferring material 16 and the material impermeable to air 17 are incorporated in the same material. Details of the different layers are as explained above. The cover has a thickness T, which is between 1 and 6 mm. Preferably, the thickness T is between 2 and 5 mm.

In one embodiment (not shown), the cover comprises a layer in which an elastic textile material and a friction increasing material are incorporated. The cover further has separate layer of a moisture absorbing and/or moisture transferring material as well as a layer of an air-tight material. Thus, in this embodiment the elastic textile material and the friction increasing material are incorporated in the same material. Details of the different layers are as explained above. The cover has a thickness T, which is between 1 and 6 mm. Preferably, the thickness T is between 2 and 5 mm.

In one embodiment (not shown), the cover comprises a layer of an elastic textile material. The elastic textile material is also a friction increasing material. The cover further has separate layer of a moisture absorbing and/or moisture transferring material as well as a layer of an air-tight material. Details of the different layers are as explained above. The cover has a thickness T, which is between 1 and 6 mm. Preferably, the thickness T is between 2 and 5 mm.

The elastic textile material 13 of a cover 10, 20, 30 as disclosed herein has a controlled elasticity, which forms a cover with compressive properties. This in turn enables the compression of soft parts of the amputation stump. When the soft parts are compressed, the load of the prosthetic socket is more evenly distributed around the amputation limb, which decreases the risk of abrasions and wounds. It will also make it overall more comfortable to wear the prosthesis.

The elastic textile material 13 preferably has an elasticity of between 10% and 600%, such as 50% to 550%, such as 100% to 500%, such as 150% to 450%, such as 200% to 425%, and most preferred 300% to 400%.

Preferably, the elastic textile material 13 is acrylic, alpaca, angora, bamboo, cotton, elastane, lycra, hemp, modal, nylon, a polyester (such as Coolmax), and/or a wool (such as Merino Wool or cashmere wool).

Further, the elastic textile material 13 may be a textile, a fabric, a woven material, such as a silicone woven material made of silicone threads, or non-woven material.

The elastic textile material 13 may be air permeable and moisture transferring.

The friction increasing material 14 may be selected from a silicone, a rubber, a gel or polyurethane (PUR).

The friction increasing material 14 is configured to increase the friction between the skin of the stump and a cover as disclosed herein. Hence, the friction increasing material 14 is arranged to be in contact with the skin surface of the amputation stump. This is achieved either by arranging the friction increasing material 14 on the interior surface 15 of the cover as disclosed herein (e.g. as seen in Fig. 1c and 1d) or by incorporating the friction increasing material 14 into the elastic textile material 13 (e.g. as shown in Fig. 1a and 1b). Friction between the skin of the amputation stump and the cover 10a, 10b, 10c, 10d causes the cover 10a, 10b, 10c, 10d to stay in place during use. When the cover 10a, 10b, 10c, 10d stays put, the risk of sores caused by the socket of the prosthesis is minimized. As described above, the friction increasing material may be incorporated into the moisture absorbing and/or transferring material 16.

The moisture absorbing and/or moisture transferring material 16 may alternatively be arranged as a separate layer 16 as shown in Fig. 1d. The moisture absorbing and/or transferring material 16 absorbs and removes moisture from the skin surface of the amputation stump, and thus decreases the relative humidity in the environment between the stump and the socket and on the skin surface of the amputation stump.

The layer of the moisture absorbing and/or moisture transferring material 16 has a thickness of between 1 and 4 mm, preferably between 2 and 3 mm.

The layer of the moisture absorbing and/or moisture transferring material 16 should be thick enough to protect skin of the amputation limb and thin enough to avoid too high temperatures and humidity within the prosthetic member. For instance, a thicker layer of the moisture absorbing and/or moisture transferring material 16, such as 1 to 6 mm, enables more absorption of moisture and thus a more cooling effect.

The layer of the moisture absorbing and/or moisture transferring material 16 has to be thick enough to protect the stump from the hard socket.

The layer of the moisture absorbing and/or moisture transferring material 16 further has to be thin enough to fit inside the prosthesis.

Materials such as non-woven materials, wool, silica gels, textiles, fabrics, woven materials, hydrogels, super absorbents or a combination thereof may be used as the moisture absorbing and/or transferring material 16.

The use of these materials causes the relative humidity inside the prosthetic socket during use to decrease to levels as low as 50-60%. When using prosthetic covers known in the art beneath (i.e. inside) a prosthetic socket, the relative humidity often quickly, such as within only 15-20 minutes, increases to levels as high as 100%. Thus, in particularly preferred embodiments, the cover comprises a moisture absorbing material.

High relative humidity is an effect of the tight fitting between the stump and socket, and also the materials used in conventional covers for the stump known in the art. For instance, many conventional covers comprise a compressive material that is not moisture absorbing, resulting in that moisture is not absorbed properly from the stump, which causes the relative humidity adjacent to the skin to increase. Other covers are made of silicone provided with textile on an internal side facing the stump to decrease friction since the friction between the silicone and the skin is too high. Nor this solution absorbs enough moisture to make sure that the relative humidity at the skin surface is kept sufficiently low.

Human skin is sensitive to moisture, and hence, the resistance of the skin is significantly reduced at high humidity levels. However, at a relative humidity of 50-60%, the skin is much more resistant. An environment having a relative humidity of 100% is also experienced as very hot and uncomfortable, while levels at 50-60% are not perceived as such.

By decreasing the relative humidity in the environment which the skin is exposed to inside the socket, the risk of the user getting abrasions and wounds from the wear from the prosthetic socket is decreased. In addition, the experience for the user is more comfortable since the covers 10a-d, 20a-d, 30a-e, 40, 50, 60, 70a-d disclosed herein decrease the relative humidity inside the socket.

These effects may facilitate a recently amputated user to be able to use his/her prosthesis for longer periods sooner than when using covers presently available on the market. Further, decreasing the risk of formation of wounds on the stump is also important for users having additional conditions. For example, a user also suffering from diabetes are more prone to bacterial infections caused by wounds formed on the amputation stump.

The covers of the present disclosure surprisingly are covers which manage to maintain the level of relative humidity at the surface of the stump at 50-60%. In addition, the covers of the present disclosure comprise an air-tight material 17 such that a secure attachment between the stump and a prosthetic socket can be achieved.

Preferably, the moisture absorbing material is a super absorbent.

A silica gel which absorbs moisture may also be used as the moisture absorbing material.

If a natural material is more preferred, wool is preferable since it both transports and absorbs moisture.

The super absorbent may be a cross-linked acrylate copolymer, partially neutralised to its sodium salt.

The moisture absorbing and/or moisture transferring material 16 in Fig. 1d is distributed as an evenly thick layer around the cover 10.

The layer of the moisture absorbing and/or moisture transferring material 16 may also be arranged having different thicknesses in different areas of the cover 10. Different parts of the stump have different structure and hardness. For instance, a part of the stump which is hard due to the presence of bone tends to benefit from a thicker layer of the moisture absorbing and/or moisture transferring material 16, while parts of the stump being softer do not need the same thickness of the layer 16. Thus, a cover according to the present teaching may be custom made to fit each individual amputation stump.

In one embodiment the moisture absorbing and/or moisture transferring material 16 is arranged such that the thickness of the moisture absorbing material 16 at the closed end 12 of the cover 10 is thicker than the thickness of the moisture absorbing material 16 at the rest of the cover 10. If the end of the stump is sensitive to load, the thicker layer of the moisture absorbing and/or transferring material 16 at the closed end 12 of the cover 10 protects and unburdens the stump.

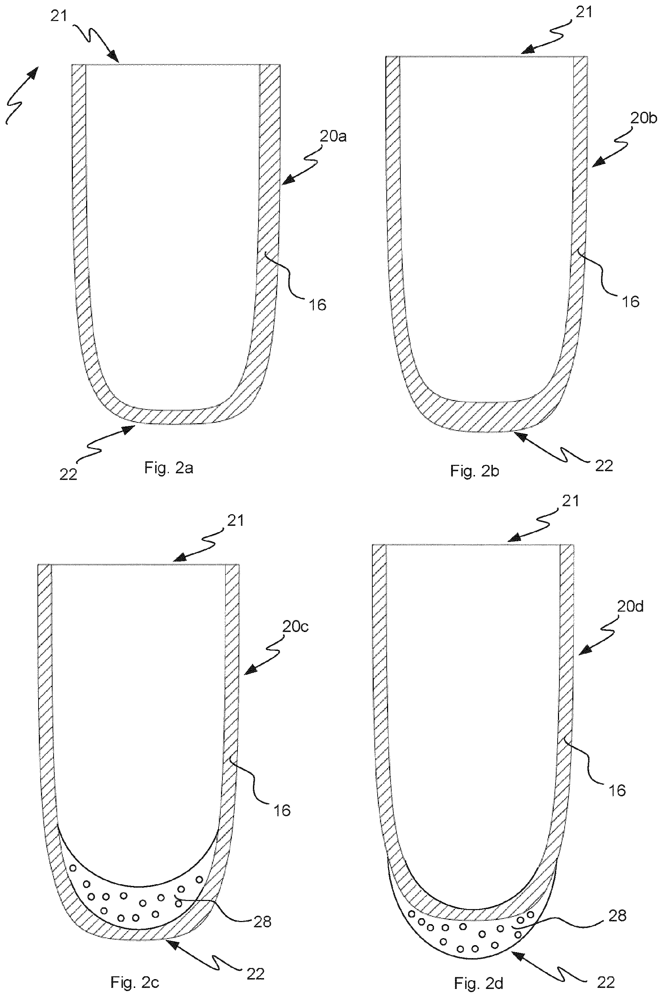

Figs. 2a-d show lateral cross sections of covers 20a-d with exemplary arrangements of the moisture absorbing and/or transferring material 16 and with exemplary arrangements of a cushioning material 28.

For simplicity, in Figs 2a-d only the moisture absorbing and/or transferring material 16 is shown. However, the skilled person realises that the covers 20a-d may comprise different combinations of different layers as explained in relation to Fig.1 or in the text above.

The covers 20a-d may comprise an elastic textile material 13, which is also a moisture absorbing and/or a moisture transferring material as explained in relation to Fig 1a or in the text above.

Alternatively, the covers 20a-d may comprise an elastic textile material 13, which is also a moisture absorbing and/or a moisture transferring material, and a friction increasing material 14 as explained above.

Alternatively, the covers 20a-d may comprise an elastic textile material 13, which is also a moisture absorbing and/or a moisture transferring material, and a layer of an air-tight material 17 as explained in relation to Fig 1b. If the cover 20a-d is used together with a prosthesis using a vacuum fastening system, it is important to prohibit air from entering in between the cover and the socket of the prosthesis. When the cover 20a-d comprises the air-tight material 17, the risk of air penetrating in between the cover and the socket is reduced and the prosthesis is securely fastened thanks to the cover 20a-d.

Alternatively, the covers 20a-d may comprise an elastic textile material 13, which is also a moisture absorbing and/or a moisture transferring material, a layer of an air-tight material 17 and a friction increasing material 14 as explained in relation to Fig 1c.

Alternatively, the covers 20a-d may comprise a layer of an elastic textile material 13, a layer of a friction increasing material 14 arranged on an interior surface 15 of the cover 20a-d and a separate layer of a moisture absorbing and/or moisture transferring material 16. Details of the different layers are as explained above.

Alternatively, the covers 20a-d may comprise an elastic textile material 13 and a separate layer of a moisture absorbing and/or moisture transferring material 16. Details of the different layers are as explained above.

Alternatively, the covers 20a-d may comprise an elastic textile material 13, a separate layer of a moisture absorbing and/or moisture transferring material 16, and a separate friction increasing material 14 and a layer of an air-tight material 17 as explained in relation to Fig 1d.

Alternatively, the covers 20a-d comprise an elastic textile material 13, a moisture absorbing and/or moisture transferring material 16, a friction increasing material 14 and an air-tight material 17, distributed between two, three or four layers forming the cover 20a-d as explained above.

Fig. 2a shows a lateral cross section of a cover 20a with an exemplary arrangement of the moisture absorbing and/or transferring material 16. The cover 20a has an open end 21 and a closed end 22. The moisture absorbing and/or transferring material 16 in Fig. 2a is thicker at the right side than at the left side of the cover 20a.

Fig. 2b presents another embodiment of a cover 20b, having an open end 21 and a closed end 22 opposite said open end 21. The cover 20b further comprises the moisture absorbing and/or transferring material 16 provided as a thicker layer at the closed end 22 of the cover 20b. The thicker layer of the moisture absorbing and/or transferring material 16 moderates the load and pressure between the residual limb and the prosthesis. This provides a more comfortable wear for the amputee and reduces the risk of abrasions on the end of the amputation stump.

The cover may be provided with a cushion formed of a cushioning material 28 at the closed end 22 of the cover 20 (Figs 2c and 2d). Similar to the thicker layer of the moisture absorbing and/or transferring material 16 in Fig. 2b, the cushion moderates the load between the residual limb and the prosthesis. This provides a more comfortable wear for the amputee and reduces the risk of abrasions on the end of the amputation stump. In addition, patients having very sensitive stumps or being recently amputated benefit from the cushioning material 28 since it relieves stress and provides comfort for the stump.

The cushioning material 28 may be for instance the same material as the moisture absorbing and/or moisture transferring material 16. In such case, the cushion may be arranged as a thicker layer of the moisture absorbing and/or moisture transferring material 16 at the closed end 22 of the cover 20 (corresponding to an embodiment resembling the embodiment shown in Fig 2b).

The cushioning material 28 may be a silicone, a gel, a rubber or any other pressure relieving materials.

The cushioning material 28 may be arranged at the closed end 22 and on the inside surface of the cover 20c (Fig. 2c).

Alternatively, the cushioning material 28 may be arranged at the closed end 22 and on the outside surface of the cover 20d (Fig. 2d).

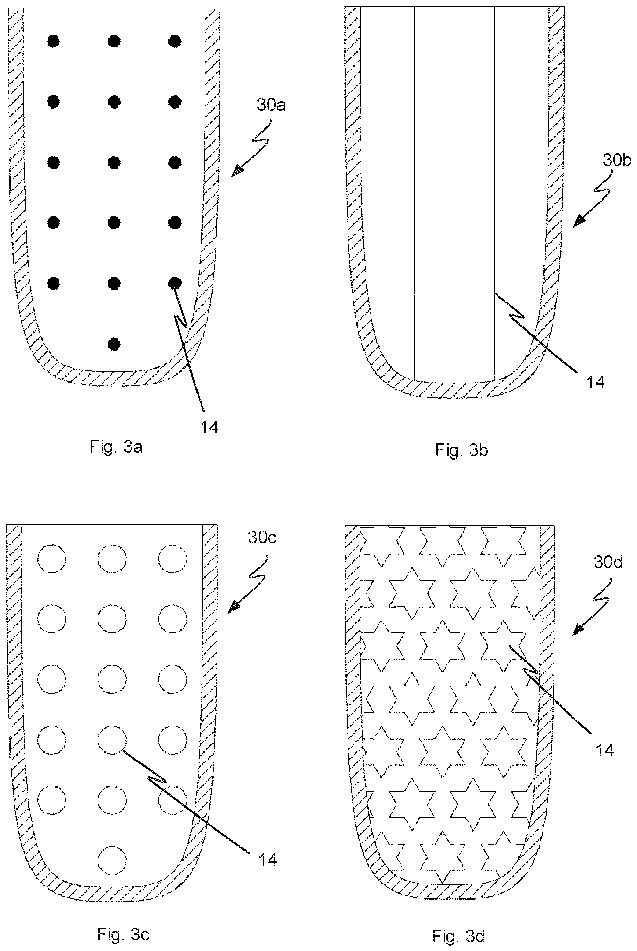

Figs. 3a-e show lateral cross sections of covers 30a-e with exemplary arrangements of the friction increasing material 14.

As shown in Figs. 3a-3e, the friction increasing material 14 may be arranged as stripes, dots, rings figures and/or circumferential rings on the inside of the cover.

The friction increasing material 14 may be arranged as a combination of these different geometrical shapes or any other arrangement causing the friction increasing material 14 to be in contact with the skin of the amputation stump.

In Fig. 3a, the friction increasing material 14 is arranged as dots distributed on the interior surface of the cover 30a.

For simplicity, in Figs 3a-d only the moisture absorbing and/or transferring material 16 is shown.

The friction increasing material 14 may be arranged as lines or stripes as shown in the cover 30b in Fig. 3b.

The cover 30c shown in Fig. 3c comprises the friction increasing material 14 in the shape of circles.

The cover 30d in Fig. 3d comprises the friction increasing material 14 in the form of star-shaped figures.

The skilled person realizes that the friction increasing material 14 can have any geometrical shape.

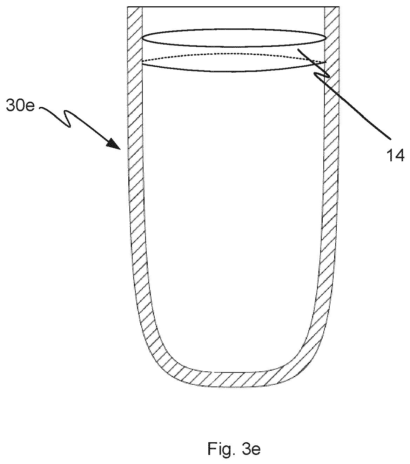

The cover 30e in Fig. 3e comprises the friction increasing material 14 in the form of circumferential rings on the inside of the cover 30e.

In addition, these different arrangements may be combined. For instance, in the covers 30a, 30b, 30c and 30d a circumferential ring of the friction increasing material 14 may be provided on the interior surface close to the open end 31 of the cover 30a, 30b, 30c and 30d (not shown).

The friction increasing material 14 may also be incorporated in the elastic textile material 13, forming a joint layer in contact with the skin of the stump.

The friction increasing material 14 may also be incorporated in the moisture absorbing and/or a moisture transferring material 16, forming a joint layer in contact with the skin of the stump.

Alternatively, the covers 30a-e may comprise an elastic textile material 13, which is also a moisture absorbing and/or a moisture transferring material, a layer of an air-tight material 17 and a friction increasing material 14 as explained in relation to Fig 1c.

Alternatively, the covers 30a-e may comprise an elastic textile material 13, a separate layer of a moisture absorbing and/or moisture transferring material 16, and a separate friction increasing material 14 and a layer of an air-tight material 17 as explained in relation to Fig 1d.

Alternatively, the covers 30a-e comprise an elastic textile material 13, a moisture absorbing and/or moisture transferring material 16, a friction increasing material 14 and an air-tight material 17, distributed between two, three or four layers forming the cover 30a-e.

Independent of the number and nature of the layers 13, 14, 16 and 17 present in a cover according to the present teaching, the exemplary arrangements of the moisture absorbing and/or transferring material 16 and with exemplary arrangements of a cushioning material 28 as described above may be combined with one or more of the exemplary arrangements of the friction increasing material 14 as shown in Figs 3a-e and described above.

Hence, the cover according to the present teaching preferably comprises an elastic textile material 13, a friction increasing material 14, a moisture absorbing and/or moisture transferring material 16 and an air-tight material 17 present in a number of layers which together forms the cover 10, 20, 30, 40, 50, 60, 70.

For instance, the elastic textile material 12 may also be a friction increasing material 14, and the moisture absorbing and/or moisture transferring material 16 may also be an air-tight material 17.

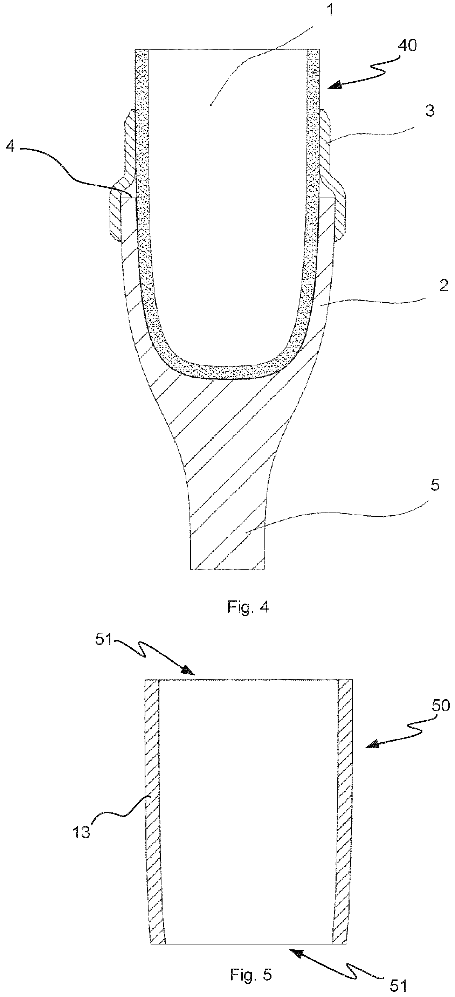

Fig. 4 shows how an amputation stump 1 covered with a cover 40 according to the present teaching is arranged in a prosthetic socket 2 of a prosthetic device 5, which e.g. may be a prosthetic leg.

The socket 2 is hard and the cover 40 thus decreases the wear between the skin surface of the amputation stump 1 and an internal surface of the hard socket 2.

The arrangement shown in Fig. 4 further comprises a sleeve 3 covering an upper edge 4 of the socket 2. The sleeve 3 is used to form an additional seal to prevent air from penetrating into the socket 2. A sleeve 3 is not always used but is common when using lower leg prostheses.

The cover 40 in Fig. 4 is any cover as disclosed herein. Thus, the cover 40 may consist of the layers 13, 14, 16 and 17 as described above. Further, the cover 40 may comprise any of the exemplary arrangements of the moisture absorbing and/or transferring material 16. Moreover, the cover 40 may comprise any exemplary arrangements of a cushioning material 28 as described above. Finally, the cover 40 may comprise one or more of the exemplary arrangements of the friction increasing material 14 as described above.

A cover according to the present teaching may also be in the form of a sleeve 50. A lateral cross section of one embodiment of such a sleeve 50 is shown in Fig. 5. The sleeve (cover) 50 in Fig. 5 comprises two open ends 51, 51 opposite to each other. Thus, the sleeve 50 may be threaded onto an amputation stump and enclose for instance a knee or an elbow.

The sleeve 50 may have different diameters along its length. For example, one open end 51 may have a width or diameter smaller than the diameter of the other open end 51 of the sleeve. Thus, the sleeve 50 may have a shape similar to a truncated cone. Preferably, the proximal end, i.e. the end intended to be closest to the torso of the patient, of the sleeve 50 is wider (has larger diameter) than the distal end, i.e. the end intended to be closest to the end of the amputation stump, of the sleeve 50. The skilled person realizes that the exact dimensions of the sleeve 50 preferably are individually adjusted to fit the amputation stump.

The sleeve 50 may be used to cover the upper edge 4 of the socket 2, as shown in Fig. 4. If the cover is used together with a prosthesis using a vacuum fastening system or a suction suspension system, the sleeve 50 can prevent air from entering into the socket of the prosthesis.

In one embodiment, the sleeve 50 comprises a layer of an elastic textile material 13, which also is a moisture absorbing and/or transferring material. The sleeve 50 may comprise the same materials as the covers 10a-d and in all the different arrangements disclosed herein.

For simplicity, in Fig. 5 only the elastic textile material 13 is shown. However, the skilled person realises that the sleeve 50 may comprise different combinations of different layers as explained in relation to Fig.1.

The sleeve 50 may comprise an elastic textile material 13, which is also a moisture absorbing and/or a moisture transferring material as explained in relation to Fig 1a.

Alternatively, the sleeve 50 may comprise an elastic textile material 13, which is also a moisture absorbing and/or a moisture transferring material, and a friction increasing material 14 as explained above.

Alternatively, the sleeve 50 may comprise an elastic textile material 13, which is also a moisture absorbing and/or a moisture transferring material, a layer of an air-tight material 17 and a friction increasing material 14 as explained in relation to Fig 1c.

Alternatively, the sleeve 50 may comprise a layer of an elastic textile material 13, a layer of a friction increasing material 14 arranged on an interior surface 15 of the sleeve 50 and a separate layer of a moisture absorbing and/or moisture transferring material 16. Details of the different layers are as explained above.

Alternatively, the sleeve 50 may comprise an elastic textile material 13 and a separate layer of a moisture absorbing and/or moisture transferring material 16. Details of the different layers are as explained above.

Alternatively, the sleeve 50 may comprise an elastic textile material 13, a separate layer of a moisture absorbing and/or moisture transferring material 16, and a separate friction increasing material 14 and a layer of an air-tight material 17 as explained in relation to Fig 1d.

Generally, the friction increasing material is located on the inside surface, i.e. the surface of the sleeve intended to be in contact with the upper edge of a socket.

The friction increasing material 14 and the elastic textile material 13 may be incorporated in a common layer.

The friction increasing material 14 and the moisture absorbing and/or moisture transferring material 16 may be incorporated in a common layer.

Alternatively, the sleeve 50 comprises an elastic textile material 13, a moisture absorbing and/or moisture transferring material 16, a friction increasing material 14 and an air-tight material 17, distributed between two, three or four layers forming the sleeve 50.

Independent of the number and nature of the layers 13, 14, 16 and 17 present in a sleeve 50 according to the present teaching, the exemplary arrangements of the moisture absorbing and/or transferring material 16 and with exemplary arrangements of a cushioning material 28 as described above may be combined with one or more of the exemplary arrangements of the friction increasing material 14 as shown in Figs 3a-e and described above.

The friction increasing material 14 may be more densely spread on the interior surface 15 at the end parts of the sleeve 50.

In one embodiment, the sleeve 50 comprises a friction increasing material 14 covering the internal upper and lower edges of the sleeve 50 (not shown) and further comprises an outer layer of an air-tight material 17 (not shown).

As mentioned above, the covers disclosed herein may be used together with a prosthesis that is fastened to the amputation stump using various kinds of fastening systems.

The cover may be used together with a vacuum or suction suspension system. In such case, the amputation stump covered with a cover comprising an air-tight material according to the present teaching is introduced into a socket of a prosthetic member and vacuum is established in the socket causing the amputation stump to be firmly connected to the prosthesis.

Vacuum may be accomplished either passively or actively. Passive vacuum is achieved if the amputation stump fits perfectly into the prosthetic socket. When the stump is introduced into the socket, air is removed from the socket through a one-way exhaust air valve and vacuum is formed inside the socket. A cover according to the present teaching is rolled onto an amputation stump and is inserted into the socket of a prosthesis. When the amputation stump covered with the cover is inserted into the socket and when the body weight of the amputee is applied as the amputee stands up, air contained in the socket permeates through the one-way exhaust valve. A suction is created within the socket, which provides an even adhesion between the cover and the socket.

If vacuum is to be formed actively, the prosthetic socket comprises some type of air pump, such as a mechanical pump which may be operated manually, or an electric pump.

Vacuum improves the adherence between the socket and the amputation stump, which provides a comfortable fit. A cover comprising an air-tight material according to the present teaching is especially suitable for such use.

A sleeve 3 may also be used in a vacuum fastening system, which creates a seal around the upper edge 4 of the socket 2, which further reduces the risk of air penetrating in between the cover and the prosthetic socket.

Alternatively, the cover may be used with a so called lanyard system. In such case, the cover comprises a first strap near and proximal to the top open end 11, 21, 31, 41, 51, 61, 71 of the cover, and a second strap at the bottom closed end 12, 22, 32, 42, 62, 72 of the cover. The cover is fitted onto the amputation stump. Further, the socket of the prosthesis in the lanyard system comprises a first slit through which the first strap may be introduced from the inside of the socket to the outside. The first strap also comprises a loop, which is thus moved through the slit and is arranged on the outside of the socket once the first strap has been pulled through the first slit. The second strap comprises at a portion close to the cover a first part of a hook-and-loop system and at a second end a second part of a hook-and-loop system. The bottom of the socket comprises a second slit where the second strap is inserted from the inside to the outside of the socket. When the second strap has been pulled through the second slit, it is threaded through the loop of the first strap. Subsequently, the two ends of the second strap may attach to each other whereupon the two parts of the hook-and-loop system engage with each other. The prosthesis has now been fastened to the amputation stump and the cover protects the stump from abrasions and too high humidity during wear.

A lanyard system may also be arranged with a single strap in the bottom closed end of the cover, which is pulled through a hole in the bottom of the prosthetic socket and subsequently securely fastened in for instance a loop, ring or hook on the outer side of the prosthetic socket. The strap may be threaded through the loop, ring or hook and secured to itself by any suitable fastening means.

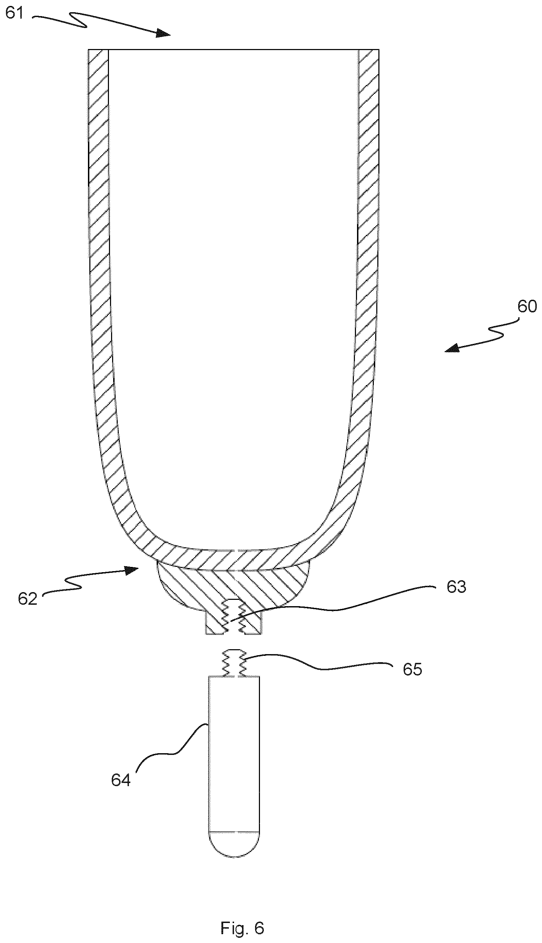

The cover may also be used together with a pin and lock system, such as a shuttle lock system. Such a cover 60 is shown in Fig. 6. The cover 60 comprises an open end 61 and a female threaded portion 63 arranged at a closed end 62 of the cover 60. A pin 64 comprises a male threaded portion 65. The pin is thus removably connectable to the cover 60. The cover 60 is mounted onto an amputation stump and the male threaded portion 65 of the pin 64 is screwed into the female threaded portion 63. The amputation stump covered with the cover 60 is then inserted into a prosthetic socket having a lock in its bottom (not shown). The pin 64 engages with the lock to secure the fastening of the cover 60 in the prosthetic socket (not shown).

Optionally, a strap may be attached to the pin. The strap is intended to guide the pin into the shuttle lock comprised in the socket of the prosthesis (not shown).

As exemplified above, a cover according to the present teaching is compatible with different prosthesis suspensions, such as e.g. the lanyard system, a pin and lock system, a vacuum system or a suction system.

If a cover according to the present teaching is used together with a prosthesis using a vacuum fastening system, it is important to prohibit air from entering into the socket of the prosthesis. This may be accomplished by a sleeve as explained above and/or a seal member located on the outside of the cover.

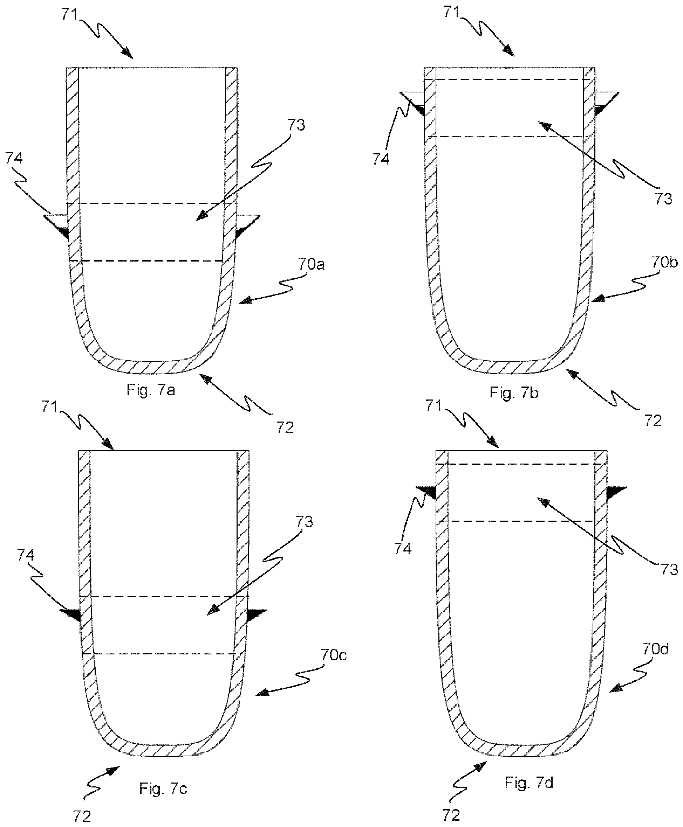

In Fig. 7a-7d, a cross section of the cover 70 is shown. The cover 70 according to the present teaching is shown together with a seal member 73. The cover 70 comprises an open end 71 and a closed end 72. The seal member 73 comprises an annular flange 74 extending from the cover 70. In some embodiments the seal member 73 comprises more than one flange 74, such as two, three or four flanges.

The seal member 73 may be made from any material suitable for sealing, such as silicone, rubber or other materials impermeable to air, such as a polymer or a co-polymer.

The flange 74 may be of variating width and thickness. The width of the seal member 73 is indicated by the dashed lines in Figs 7a-7d. The width may be 2 to 15 cm, such as 5 to 10 cm, preferably approximately 7 cm.

The seal member 73 may be a separate part, which is threaded onto the cover 70.

Alternatively, the seal member 73 is a part of the cover 70 attached to the outside of the cover 70.

The seal member 73 shown in Figs 7a-7d is used to enable the formation of vacuum between the prosthetic socket and the cover 70 and to create a seal between the cover 70 and a prosthetic socket. The flange 74 aids the formation of vacuum between the cover 70 and the socket and also prevents air from entering in between the cover 70 and the socket during use.

If the seal member 73 is part of the cover 70, the cover 70 with the seal member 73 is rolled onto the amputation stump. Subsequently, the stump wearing the cover 70 with the seal member 73 is pushed into the prosthetic socket. The flange 74 aids the formation of vacuum between the cover 70 and the socket and also prevents air from entering in between the cover 70 and the socket during use.

If a separate seal member 73 is used, the cover 70 is rolled onto the amputation stump. Then, the seal member 73 is threaded onto the outside of the cover 70. Subsequently, the stump wearing the cover 70 and the seal member 73 is pushed into the prosthetic socket. The flange 74 aids the formation of vacuum between the cover 70 and the socket and also prevents air from entering in between the cover 70 and the socket during use.

### Specific Examples of covers according to the present disclosure

*Example I:* The cover comprises four layers. The first layer is intended to have direct contact with an amputation stump and comprises a friction increasing material. The second layer covers the first layer and comprises an elastic textile material. The third layer comprises a moisture absorbing and moisture transferring material and covers the second layer. The fourth layer comprises a material impermeable to air and covers the third layer.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

*Example II:* The cover comprises four layers. The first layer is intended to have direct contact with an amputation stump and comprises a friction increasing material. The second layer covers the first layer and comprises an elastic textile material. The third layer comprises a moisture absorbing material and covers the second layer. The fourth layer comprises a material impermeable to air and covers the third layer.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

*Example III:* The cover comprises three layers. The first layer is intended to have direct contact with an amputation stump and comprises a friction increasing material. The second layer covers the first layer and comprises an elastic textile material. The third layer comprises a moisture absorbing and moisture transferring material and a material impermeable to air and covers the second layer.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

*Example IV:* The cover comprises three layers. The first layer is intended to have direct contact with an amputation stump and comprises a friction increasing material. The second layer covers the first layer and comprises an elastic textile material. The third layer comprises a moisture absorbing material and a material impermeable to air and covers the second layer.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

*Example V:* The cover comprises two layers. The first layer is intended to have direct contact with an amputation stump and comprises a friction increasing material and an elastic textile material. The second layer covers the first layer and comprises a moisture absorbing and moisture transferring material and a material impermeable to air.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

*Example VI:* The cover comprises two layers. The first layer is intended to have direct contact with an amputation stump and comprises a friction increasing material and an elastic textile material. The second layer covers the first layer and comprises a moisture absorbing material and a material impermeable to air.

The friction increasing material is preferably chosen from silicone, rubber and a soft plastic.

The elastic textile material is preferably chosen from silicone, rubber and polyurethane.

The moisture absorbing and moisture transferring material is preferably chosen from wool, cotton and super absorbent material.

The material impermeable to air is preferably a solid hydrophilic plastic.

The cover may further have a seal with 1 to 3 flanges, preferably 1 flange.

### Reference Signs

- 1: amputation stump
- 2: prosthetic socket
- 3, 50: sleeve
- 4: upper edge of a prosthetic socket
- 10a-d, 20a-d, 30a-3, 40, 50, 60, 70: cover
- 11, 21, 31, 51, 61, 71: open end
- 12, 22, 32, 52, 62, 72: closed end
- 13: elastic textile material
- 14: friction increasing material
- 15: interior surface of the cover
- 16: moisture absorbing and/or moisture transferring material
- 17: air-tight material
- 28: cushioning material
- 63: female threaded portion
- 64: pin
- 65: male threaded portion
- 73: seal member
- 74: flange on seal member

## Claims

1. A tube-shaped cover (10, 20, 30, 40, 50, 60, 70) for covering an amputation stump (1), wherein the cover has at least one open end (11, 21, 31, 41, 51, 61, 71) for introduction of said amputation stump (1), wherein the cover (10, 20, 30, 40, 50, 60, 70) comprises:
- an elastic textile material (13) in the shape of a tube;
- a friction increasing material (14) arranged to be in contact with the amputation stump (1); and
- a moisture absorbing and/or moisture transferring material (16);
**characterised in that** the tube-shaped cover further comprises:
- a material impermeable to air (17), wherein the material impermeable to air (17) constitutes an outer layer of the cover, wherein the material impermeable to air (17) covers the elastic textile material (13) or the moisture absorbing and/or moisture transferring material (16).

2. The cover according to claim 1, wherein the moisture absorbing and/or moisture transferring material (16) is a moisture absorbing material.

3. The cover according to claim 1 or 2, wherein the cover has two open ends (51, 51).

4. The cover according to claim 1 or 2, wherein the cover has one open end (11, 21, 31, 41, 61, 71) for introduction of said amputation stump and one closed end (12, 22, 32, 42, 62, 72) opposite said open end (11, 21, 31, 41, 61, 71).

5. The cover according to any one of the preceding claims, wherein the elastic textile material (13), the moisture absorbing and/or moisture transferring material (16), and the friction increasing material (14) are distributed between one, two or three layers forming the cover (10, 20, 30, 40, 50, 60, 70).

6. The cover according to any one of the preceding claims, wherein two or three of the elastic textile material (13), the friction increasing material (14), and the moisture absorbing and/or moisture transferring material (16) are incorporated in the same material.

7. The cover according to any one of the preceding claims, wherein the friction increasing material (14) is incorporated in the elastic textile material (13).

8. The cover according to any one of the preceding claims, wherein the moisture absorbing and/or moisture transferring material (16) is incorporated in the elastic textile material (13).

9. The cover according to any one of the preceding claims, wherein the elastic textile material (13) is a textile, a fabric, a woven material, such as a silicone woven material made of silicone threads, or a non-woven material.

10. The cover according to any one of claims 1 to 9, wherein the material impermeable to air (17) is a type of plastic, preferably a hydrophilic copolymer.

11. The cover according to any one of the preceding claims, wherein the cover is configured to be used together with a prosthetic socket (2), which is to be suspended to an amputation stump (1) by a vacuum created between the cover (10, 20, 30, 40, 50, 60, 70) and the socket (2) when the amputation stump is introduced into said cover (10, 20, 30, 40, 50, 60, 70).

12. The cover according to any one of claims 4 to 11, wherein the moisture absorbing and/or moisture transferring material (16) is arranged around the elastic textile material (13), wherein a thickness of the moisture absorbing material and/or moisture transferring material (16) at the closed end (12, 22, 32, 42, 62, 72) of the cover (10, 20, 30, 40, 50, 60, 70) is thicker than a thickness of the moisture absorbing material at the rest of the cover.

13. The cover according to any one of claims 4 to 11, further comprising a cushioning material (28) at the closed end (12, 22, 32, 42, 62, 72) of the cover (10, 20, 30, 40, 60, 70) configured to moderate the load between the amputation stump and a prosthetic socket.

14. The cover according to any one of the claims 4 to 13, further comprising a threaded portion (63) arranged at the closed (62) end of the cover (60), wherein the threaded portion (63) is adapted for the fastening of:
- a pin (64) configured to engage in a lock in a prosthetic socket, and optionally a strap configured to guide the pin (64) into the lock; or
- a strap configured to engage in a lock in a prosthetic socket.

15. The cover according to any one of claims 4 to 13, wherein the cover further comprises:
- a strap arranged proximal to the closed end of said cover,
wherein the strap is configured to guide an amputation stump covered by said cover into a prosthetic socket.

16. The cover according to any one of the preceding claims, wherein the thickness of the cover (10, 20, 30, 40, 50, 60, 70) is between 1 and 10 mm, such as 1.25 and 8 mm, such as 1.5 and 6 mm, preferably 2-5 mm.

17. A kit comprising a cover according to any one of the claims 4 to 16 and a sleeve (3, 50) configured to create a seal between an upper edge (4) of a prosthetic socket (2) and an amputation stump (1) covered by said cover (10, 20, 30, 40, 50, 60, 70).

## Patentansprüche

1. Schlauchförmige Abdeckung (10, 20, 30, 40, 50, 60, 70) zum Abdecken eines Amputationsstumpfes (1), wobei die Abdeckung wenigstens ein offenes Ende (11, 21, 31, 41, 51, 61, 71) zum Einführen besagten Amputationsstumpfes (1) aufweist, wobei die Abdeckung (10, 20, 30, 40, 50, 60, 70) umfasst:
- ein elastisches Textilmaterial (13) in der Form eines Schlauches;
- ein reibungserhöhendes Material (14), das so angeordnet ist, dass es mit dem Amputationsstumpf (1) in Kontakt kommt; und
- ein feuchtigkeitsabsorbierendes und/oder feuchtigkeitsabführendes Material (16);
**dadurch gekennzeichnet, dass** die schlauchförmige Abdeckung weiter umfasst:
- ein für Luft undurchlässiges Material (17), wobei das für Luft undurchlässige Material (17) eine äußere Schicht der Abdeckung darstellt, wobei das für Luft undurchlässige Material (17) das elastische Textilmaterial (13) oder das feuchtigkeitsabsorbierende und/oder feuchtigkeitsabführende Material (16) überdeckt.

2. Abdeckung nach Anspruch 1, wobei das feuchtigkeitsabsorbierende und/oder feuchtigkeitsabführende Material (16) ein feuchtigkeitsabsorbierendes Material ist.

3. Abdeckung nach Anspruch 1 oder 2, wobei die Abdeckung zwei offene Enden (51, 51) aufweist.

4. Abdeckung nach Anspruch 1 oder 2, wobei die Abdeckung ein offenes Ende (11, 21, 31, 41, 61, 71) zum Einführen besagten Amputationsstumpfes und ein geschlossenes Ende (12, 22, 32, 42, 62, 72) gegenüber besagten offenen Endes (11, 21, 31, 41, 61, 71) aufweist.

5. Abdeckung nach einem der vorangehenden Ansprüche, wobei das elastische Textilmaterial (13), das feuchtigkeitsabsorbierende und/oder feuchtigkeitsabführende Material (16) und das reibungserhöhende Material (14) zwischen ein, zwei oder drei Lagen verteilt sind, die die Abdeckung (10, 20, 30, 40, 50, 60, 70) bilden.

6. Abdeckung nach einem der vorangehenden Ansprüche, wobei zwei oder drei von dem elastischen Textilmaterial (13), dem reibungserhöhenden Material (14) und dem feuchtigkeitsabsorbierenden und/oder feuchtigkeitsabführenden Material (16) im selben Material eingearbeitet sind.

7. Abdeckung nach einem der vorangehenden Ansprüche, wobei das reibungserhöhende Material (14) im elastischen Textilmaterial (13) eingearbeitet ist.

8. Abdeckung nach einem der vorangehenden Ansprüche, wobei das feuchtigkeitsabsorbierende und/oder feuchtigkeitsabführende Material (16) im elastischen Textilmaterial (13) eingearbeitet ist.

9. Abdeckung nach einem der vorangehenden Ansprüche, wobei das elastische Textilmaterial (13) ein Textil, ein Stoff, ein gewebtes Material, wie etwa ein gewebtes Silikonmaterial, hergestellt aus Silikonfäden, oder ein Vliesmaterial ist.

10. Abdeckung nach einem der Ansprüche 1 bis 9, wobei das für Luft undurchlässige Material (17) eine Art von Kunststoff ist, vorzugsweise ein hydrophiles Copolymer.

11. Abdeckung nach einem der vorangehenden Ansprüche, wobei die Abdeckung so konfiguriert ist, dass sie zusammen mit einem Prothesenschaft (2) verwendet wird, der angehängt werden soll an einen Amputationsstumpf (1) durch ein Vakuum, das zwischen der Abdeckung (10, 20, 30, 40, 50, 60, 70) und dem Schaft (2) geschaffen wird, wenn der Amputationsstumpf in besagte Abdeckung (10, 20, 30, 40, 50, 60, 70) eingeführt ist.

12. Abdeckung nach einem der Ansprüche 4 bis 11, wobei das feuchtigkeitsabsorbierende und/oder feuchtigkeitsabführende Material (16) um das elastische Textilmaterial (13) herum angeordnet ist, wobei eine Dicke des feuchtigkeitsabsorbierenden Materials und/oder feuchtigkeitsabführenden Materials (16) am geschlossenen Ende (12, 22, 32, 42, 62, 72) der Abdeckung (10, 20, 30, 40, 50, 60, 70) dicker ist als eine Dicke des feuchtigkeitsabsorbierenden Materials im Rest der Abdeckung.

13. Abdeckung nach einem der Ansprüche 4 bis 11, die weiter ein Dämpfungsmaterial (28) am geschlossenen Ende (12, 22, 32, 42, 62, 72) der Abdeckung (10, 20, 30, 40, 60, 70) umfasst, das so konfiguriert ist, dass es die Last zwischen dem Amputationsstumpf und einem Prothesenschaft abmildert.

14. Abdeckung nach einem der Ansprüche 4 bis 13, die weiter einen Gewindeabschnitt (63) umfasst, der am geschlossenen (62) Ende der Abdeckung (60) angeordnet ist, wobei der Gewindeabschnitt (63) angepasst ist zur Befestigung von:
- einem Stift (64), der so konfiguriert ist, dass er in einen Verschluss in einem Prothesenschaft eingreift, und fakultativ eine Lasche, die so konfiguriert ist, dass sie den Stift (64) in den Verschluss führt; oder
- eine Lasche, die so konfiguriert ist, dass sie in einen Verschluss in einem Prothesenschaft eingreift.

15. Abdeckung nach einem der Ansprüche 4 bis 13, wobei die Abdeckung weiter umfasst:
- eine Lasche, die proximal zum geschlossenen Ende besagter Abdeckung angeordnet ist, wobei die Lasche so konfiguriert ist, dass sie einen Amputationsstumpf, der von besagter Abdeckung überdeckt ist, in einen Prothesenschaft führt.

16. Abdeckung nach einem der vorangehenden Ansprüche, wobei die Dicke der Abdeckung (10, 20, 30, 40, 50, 60, 70) zwischen 1 und 10 mm liegt, wie etwa 1,25 und 8 mm, wie etwa 1,5 und 6 mm, vorzugsweise 2 - 5 mm.

17. Satz, umfassend eine Abdeckung nach einem der Ansprüche 4 bis 16 und eine Manschette (3, 50), die so konfiguriert ist, dass sie eine Abdichtung zwischen einer oberen Kante (4) eines Prothesenschaftes (2) und einem Amputationsstumpf (1), der von besagter Abdeckung (10, 20, 30, 40, 50, 60, 70) überdeckt ist, schafft.

## Revendications

1. Manchon de forme tubulaire (10, 20, 30, 40, 50, 60, 70) destiné à recouvrir un moignon d'amputation (1), dans lequel le manchon présente au moins une extrémité ouverte (11, 21, 31, 41, 51, 61, 71) pour introduction dudit moignon d'amputation (1), dans lequel le manchon (10, 20, 30, 40, 50, 60, 70) comprend :
- un matériau textile élastique (13) sous la forme d'un tube ;
- un matériau augmentant la friction (14) disposé pour être en contact avec le moignon d'amputation (1) ; et
- un matériau absorbant l'humidité et/ou transférant l'humidité (16) ;
**caractérisé en ce que** le manchon de forme tubulaire comprend en outre :
- un matériau imperméable à l'air (17), dans lequel le matériau imperméable à l'air (17) constitue une couche externe du manchon, dans lequel le matériau imperméable à l'air (17) recouvre le matériau textile élastique (13) ou le matériau absorbant l'humidité et/ou transférant l'humidité (16).

2. Manchon selon la revendication 1, dans lequel le matériau absorbant l'humidité et/ou transférant l'humidité (16) est un matériau absorbant l'humidité.

3. Manchon selon la revendication 1 ou 2, dans lequel le manchon présente deux extrémités ouvertes (51, 51).

4. Manchon selon la revendication 1 ou 2, dans lequel le manchon présente une extrémité ouverte (11, 21, 31, 41, 61, 71) pour introduction dudit moignon d'amputation et une extrémité fermée (12, 22, 32, 42, 62, 72) en regard de ladite extrémité ouverte (11, 21, 31, 41, 61, 71).

5. Manchon selon l'une quelconque des revendications précédentes, dans lequel le matériau textile élastique (13), le matériau absorbant l'humidité et/ou transférant l'humidité (16), et le matériau augmentant la friction (14) sont répartis entre une, deux ou trois couches formant le manchon (10, 20, 30, 40, 50, 60, 70).

6. Manchon selon l'une quelconque des revendications précédentes, dans lequel deux ou trois parmi le matériau textile élastique (13), le matériau augmentant la friction (14) et le matériau absorbant l'humidité et/ou transférant l'humidité (16) sont incorporés dans le même matériau.

7. Manchon selon l'une quelconque des revendications précédentes, dans lequel le matériau augmentant la friction (14) est incorporé dans le matériau textile élastique (13).

8. Manchon selon l'une quelconque des revendications précédentes, dans lequel le matériau absorbant l'humidité et/ou transférant l'humidité (16) est incorporé dans le matériau textile élastique (13).

9. Manchon selon l'une quelconque des revendications précédentes, dans lequel le matériau textile élastique (13) est un textile, un tissu, un matériau tissé, tel qu'un matériau tissé en silicone constitué de fils de silicone, ou un matériau non tissé.

10. Manchon selon l'une quelconque des revendications 1 à 9, dans lequel le matériau imperméable à l'air (17) est un type de matière plastique, de préférence un copolymère hydrophile.

11. Manchon selon l'une quelconque des revendications précédentes, dans lequel le manchon est configuré pour être utilisé conjointement avec une emboîture prothétique (2), qui doit être suspendue à un moignon d'amputation (1) par un vide créé entre le manchon (10, 20, 30, 40, 50, 60, 70) et l'emboîture (2) lorsque le moignon d'amputation est introduit dans ledit manchon (10, 20, 30, 40, 50, 60, 70).

12. Manchon selon l'une quelconque des revendications 4 à 11, dans lequel le matériau absorbant l'humidité et/ou transférant l'humidité (16) est disposé autour du matériau textile élastique (13), dans lequel une épaisseur du matériau absorbant l'humidité et/ou transférant l'humidité (16) au niveau de l'extrémité fermée (12, 22, 32, 42, 62, 72) du manchon (10, 20, 30, 40, 50, 60, 70) est supérieure à une épaisseur du matériau absorbant l'humidité dans le reste du manchon.

13. Manchon selon l'une quelconque des revendications 4 à 11, comprenant en outre un matériau d'amortissement (28) au niveau de l'extrémité fermée (12, 22, 32, 42, 62, 72) du manchon (10, 20, 30, 40, 60, 70) configuré pour atténuer la charge entre le moignon d'amputation et une emboîture prothétique.

14. Manchon selon l'une quelconque des revendications 4 à 13, comprenant en outre une partie filetée (63) disposée au niveau de l'extrémité fermée (62) du manchon (60), dans lequel la partie filetée (63) est adaptée à la fixation :
- d'une goupille (64) configurée pour venir en prise dans un verrou d'une emboîture prothétique, et facultativement d'une sangle configurée pour guider la goupille (64) dans le verrou ; ou
- d'une sangle configurée pour venir en prise dans un verrou d'une emboîture prothétique.

15. Manchon selon l'une quelconque des revendications 4 à 13, dans lequel le manchon comprend en outre :
- une sangle disposée à proximité de l'extrémité fermée dudit manchon,
dans lequel la sangle est configurée pour guider un moignon d'amputation recouvert par ledit manchon dans une emboîture prothétique.

16. Manchon selon l'une quelconque des revendications précédentes, dans lequel l'épaisseur du manchon (10, 20, 30, 40, 50, 60, 70) est comprise entre 1 et 10 mm, telle qu'entre 1,25 et 8 mm, telle qu'entre 1,5 et 6 mm, de préférence entre 2 et 5 mm.

17. Kit comprenant un manchon selon l'une quelconque des revendications 4 à 16 et un manchon (3, 50) configuré pour créer un joint d'étanchéité entre un bord supérieur (4) d'une emboîture prothétique (2) et un moignon d'amputation (1) recouvert par ledit manchon (10, 20, 30, 40, 50, 60, 70).
